(19) Europäisches Patentamt
European Patent Office
Office européen des brevets

(11) Veröffentlichungsnummer: **0 053 693**
**A1**

(12) # EUROPÄISCHE PATENTANMELDUNG

(21) Anmeldenummer: 81108767.5

(22) Anmeldetag: 23.10.81

(51) Int. Cl.³: C 07 D 499/64
A 61 K 31/43
//C07D239/48, C07D413/12,
C07D417/12, C07D403/12,
C07D409/12

(30) Priorität: 05.12.80 DE 3045908
12.12.80 DE 3046839

(43) Veröffentlichungstag der Anmeldung:
16.06.82 Patentblatt 82/24

(84) Benannte Vertragsstaaten:
AT BE CH DE FR IT LI LU NL SE

(71) Anmelder: Dr. Karl Thomae GmbH
Postfach 1755
D-7950 Biberach (Riss)(DE)

(72) Erfinder: Maier, Roland, Dr. Dipl.-Chem.
Bodelschwinghstrasse 39
D-7950 Biberach 1(DE)

(72) Erfinder: Wetzel, Bernd, Dr. Dipl.-Chem.
Kapellenweg 21
D-7950 Biberach 1(DE)

(72) Erfinder: Woitun, Eberhard, Dr. Dipl.-Chem.
Stecherweg 17
D-7950 Biberach 1(DE)

(72) Erfinder: Reuter, Wolfgang, Dr. Dipl.-Chem.
Nelkenweg 10
D-7951 Laupertshausen(DE)

(72) Erfinder: Lechner, Uwe, Dr.
Panoramastrasse 12
D-7951 Ummendorf(DE)

(72) Erfinder: Goeth, Hanns, Dr.
Oberer Bühl 6
D-7950 Biberach 1(DE)

(54) **Neue Penicilline, ihre Salze, Verfahren zu ihrer Herstellung und diese Verbindungen enthaltende Arzneimittel.**

(57) Beschrieben werden neue Penicilline der allgemeinen Formel

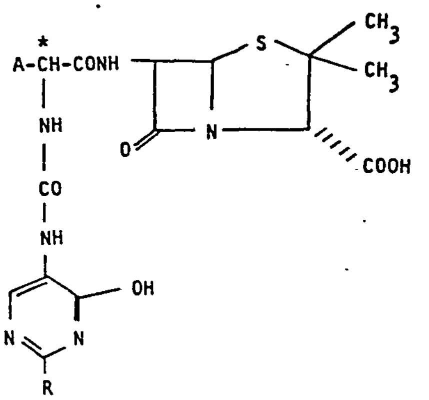

,(I)

in der A die Phenyl-, 4-Hydroxyphenyl-, 2-Thienyl- oder 3,4-Dihydroxyphenylgruppe bedeutet und R folgende Bedeutungen besitzt:

a) eine Gruppe der allgemeinen Formel IIa

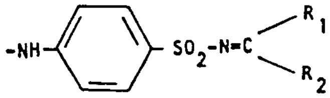

,(IIa)

in der $R_1$ und/oder $R_2$ die Methylmercapto- oder die Aminogruppe bedeuten,

b) eine Gruppe der allgemeinen Formel IIb

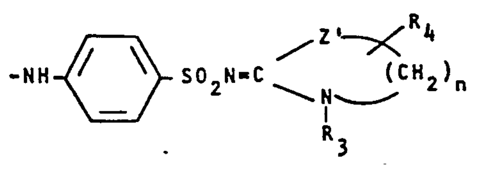

,(IIb)

in der Z' für –O–, –S– oder =$NR_3$ steht, $R_3$ ein Wasserstoffatom oder eine, gegebenenfalls durch eine Hydroxylgruppe substituierte Alkylgruppe, $R_4$ ein Wasserstoffatom, die Hydroxy-, Hydroxymethyl- oder Methylgruppe und n die Zahlen 2, 3 oder 4 darstellen,

c) eine Gruppe der allgemeinen Formel

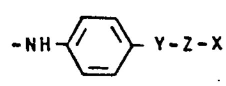

,

./...

EP 0 053 693 A1

Croydon Printing Company Ltd.

in der Y die Gruppen $-SO_2-NH-$, $-SO-$ oder $-SO_2-$ und Z eine niedere Alkylengruppe und X eine Hydroxy-, Aminocarbonyl-, Aminosulfonyl-, Formylamino-, Acetylamino-, Amino-, Methylsulfinyl-, Methylsulfonyl- oder eine Aminocarbonsäuregruppe der Formel $-CH(NH_2)COOH$ sein können oder worin $-Y-Z-X$ die Gruppen

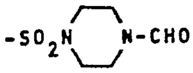, 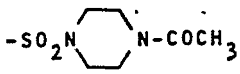, 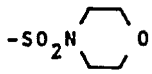,

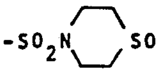, 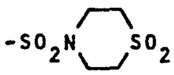, $-SO_2NHCH_2CHOHCH_2OH$ oder

$-SO-CH_2-CHOH-CH_2OH$ bedeutet und ihre physiologisch verträglichen Salze mit anorganischen oder organischen Basen.

Die Verbindungen der allgemeinen Formel I wirken sowohl gegen grampositive als auch besonders gegen gramnegative Bakterien und zeigen ein breites Wirkungsspektrum.

0053693



Case 5/819
Dr. Bu/pf

DR. KARL THOMAE GMBH, BIBERACH AN DER RISS

Neue Penicilline, ihre Salze, Verfahren zu ihrer Herstellung und diese Verbindungen enthaltende Arzneimittel

Die Erfindung betrifft neue Penicilline der allgemeinen Formel I

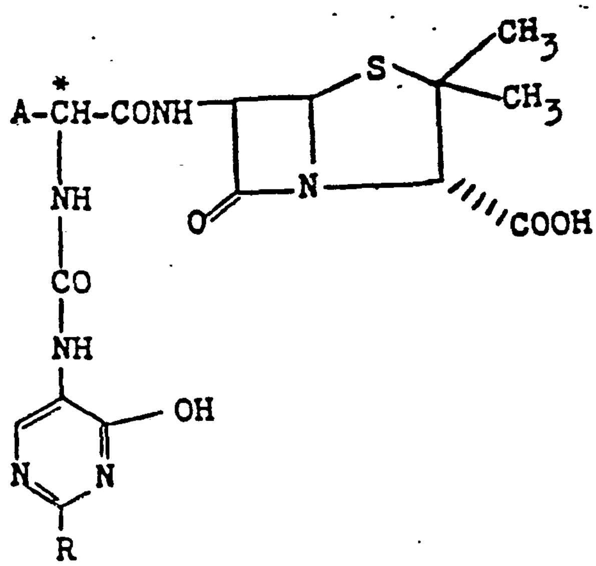

,(I)

bzw. deren Tautomere der allgemeinen Formel I'

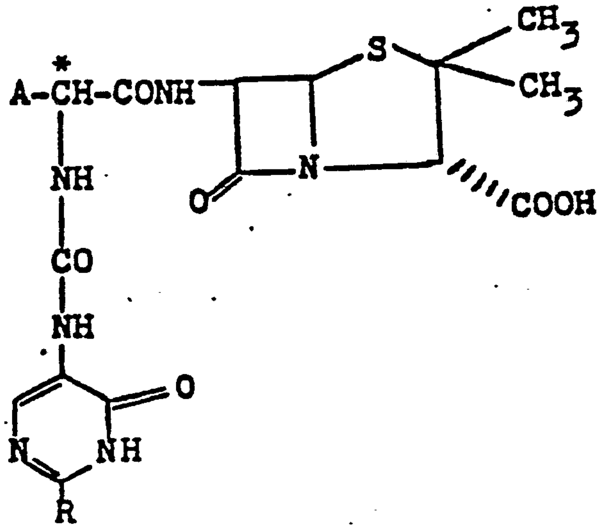

,(I')

ihre physiologisch verträglichen Salze mit anorganischen oder organischen Basen, Verfahren zur Herstellung dieser Verbindungen und diese Verbindungen enthaltende Arzneimittel.

In den obigen allgemeinen Formeln I und I' bedeutet:

A die Phenyl-, 4-Hydroxyphenyl-, die 2-Thienyl- oder die 3,4-Dihydroxyphenylgruppe und

R 1.) eine Gruppe der allgemeinen Formel IIa

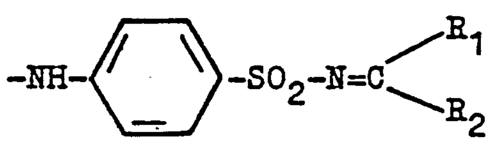

,(IIa)

in der die Reste $R_1$ und $R_2$ gleich oder verschieden sind und die Methylmercaptogruppe oder die Aminogruppe bedeuten, oder

2.) eine Gruppe der allgemeinen Formel IIb

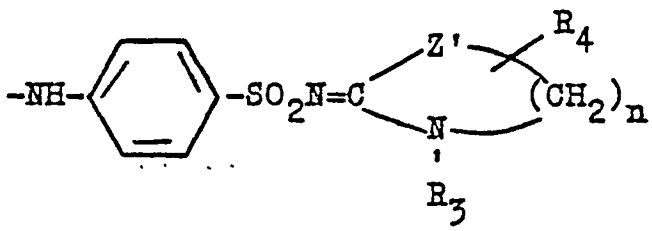 ,(IIb)

in der
Z' für -O-, -S- oder $=NR_3$ und
$R_3$ für ein Wasserstoffatom oder eine Alkylgruppe mit 1 bis 3 Kohlenstoffatomen steht, die gegebenenfalls durch eine Hydroxygruppe substituiert sein kann und
$R_4$ ein Wasserstoffatom, die Hydroxy-, Hydroxymethyl- oder Methylgruppe bedeutet
und n die Zahlen 2, 3 oder 4 darstellt, wobei die Gruppen der Formel IIa und IIb, wenn einer oder beide Reste $R_1$ und $R_2$ eine Aminogruppe bedeuten oder wenn $R_3$ Wasserstoff darstellt, auch in den folgenden tautomeren Formen der Formeln IIa' bzw. IIb' vorliegen können:

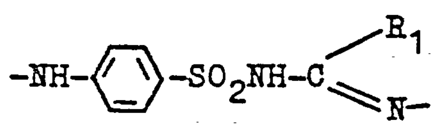 oder 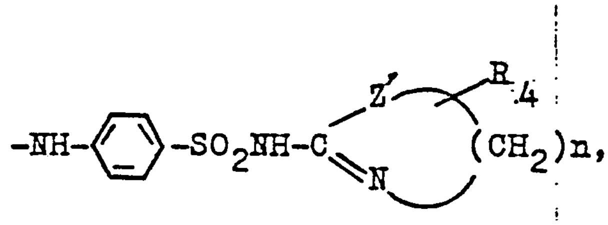

IIa' IIb'

oder
3.) eine Gruppe der allgemeinen Formel

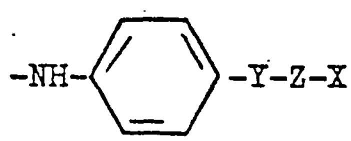

in der
Y eine Gruppe der Formel $-SO_2-NH-$, -SO- oder $-SO_2-$ und
Z eine geradkettige oder verzweigte Alkylengruppe mit 1 bis 3 Kohlenstoffatomen und
X eine Hydroxy-, Aminocarbonyl-, Aminosulfonyl-, Formylamino-, Acetylamino-, Amino-, Methylsulfinyl-, Methylsulfonyl-, oder eine Aminocarbonsäuregruppe

der Formel

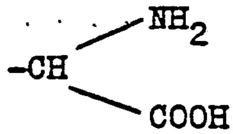

sein können,

oder es bedeutet -Y-Z-X die Gruppe $-SO_2N(CH_2CH_2OH)_2$, die Gruppe 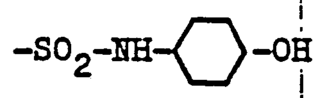

die Gruppe 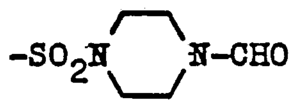 oder 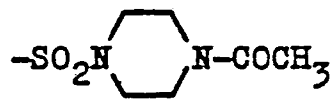,

die Gruppe 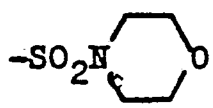, oder 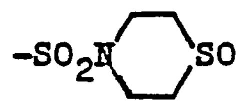 oder

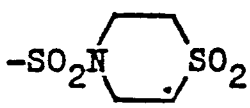, oder die Gruppe

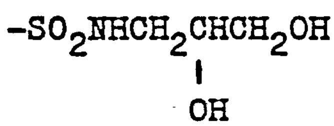 oder 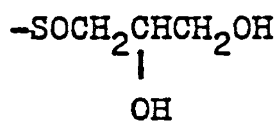.

Besonders bevorzugt sind diejenigen Verbindungen der allgemeinen Formel I bzw. I', in der A die Phenyl- oder p-Hydroxyphenylgruppe darstellt, und R für folgende Reste steht:

1.) für die Gruppe

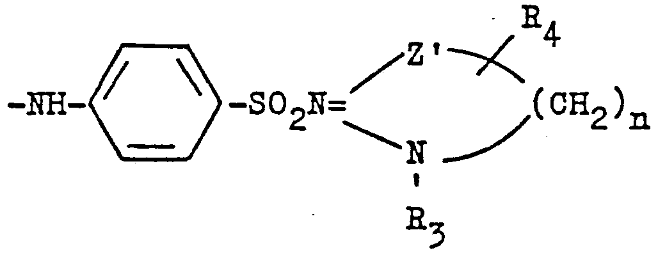,

wobei n=2, 3 oder 4 bedeutet, Z die Gruppe $=NR_3$ darstellt, und

$R_3$ und $R_4$ ein Wasserstoffatom oder die Methylgruppe bedeuten, oder

2.) für die Gruppe

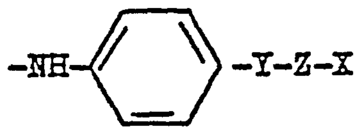

in der Y und Z die oben angegebene Bedeutung besitzen und
X für die Hydroxy-, Aminocarbonyl-, Aminosulfonyl-, Acetylamino-, Methylsulfinyl- oder die Methylsulfonylgruppe steht oder in der
-Y-Z-X eine der Gruppen

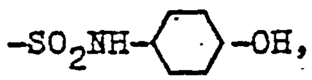, $-SO_2N(CH_2CH_2OH)_2$, 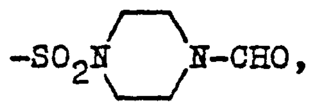,

$-SO_2NHCH_2CHCH_2OH$ oder $-SOCH_2-CHCH_2OH$ darstellt.
OH OH

Die Verbindungen der allgemeinen Formeln I bzw. I' können bezüglich des Chiralitätszentrums C+ in den beiden möglichen R- und S-Konfigurationen, jedoch auch als ein Gemisch dieser beiden Konfigurationen, vorliegen. Besonders bevorzugt sind solche Verbindungen, für welche die D=R-Konfiguration zutrifft.

Die Verbindungen der allgemeinen Formeln I bzw. I' zeigen ein breites Wirkungsspektrum gegen grampositive und gramnegative Bakterien bei einer sehr guten Verträglichkeit beim Menschen.

Die Verbindungen der allgemeinen Formeln I bzw. I' lassen sich wie folgt darstellen:

1) Durch Umsetzung einer Verbindung der allgemeinen Formel III,

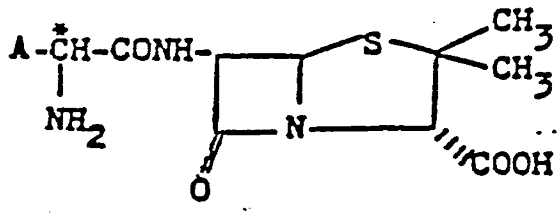

,(III)

in der A wie oben definiert ist, mit einem Pyrimidinderivat der allgemeinen Formel IV,

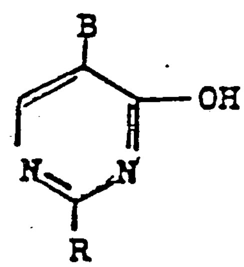

,(IV)

in der R wie oben definiert ist und B die Gruppe -NCO oder ein reaktives Derivat der Gruppe-NHCOOH bedeutet, wie z.B.

die Gruppen -NHCOCl, -NHCOBr oder -NH-COO-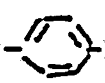-$NO_2$,

wobei die Gruppe NHCOCl besonders bevorzugt ist. Es können auch Gemische von solchen Pyrimidinderivaten der allgemeinen Formel IV verwendet werden, in der B teils die eine und teils die andere der vorstehend genannten Bedeutungen besitzt, z. B. die Gruppen -NCO und $-\underset{H}{N}COCl$ gleichzeitig nebeneinander.

Die Ausgangsprodukte der allgemeinen Formel III können in Form ihrer anorganischen oder organischen Salze, z.B. als Triäthylammoniumsalz oder Natriumsalz, eingesetzt werden. Die Reaktion kann dann in beliebigen Mischungen von Wasser mit solchen organischen Lösungsmitteln, die mit Wasser mischbar sind, wie Ketonen, z.B. Aceton, cyclische Äther, z.B. Tetrahydrofuran oder Dioxan, Nitrilen, z.B. Acetonitril, Formamiden, z.B. Dimethylformamid, Dimethylsulfoxid oder Alkoholen z.B. Isopropanol oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z.B. halogenierten Kohlenwasserstoffen, wie Chloroform oder Methylenchlorid unter Zusatz von Basen, vorzugsweise Triäthylamin, Diäthylamin oder N-Äthylpiperidin durchzuführen. Weiterhin läßt sich die Reaktion in einem Gemenge aus Wasser und einem mit Wasser nicht mischbaren Lösungsmittel, wie Äther, z.B. Diäthyläther, halogenierten Kohlenwasserstoffen, z.B. Chloroform oder Methylenchlorid, Schwefelkohlenstoff, Ketonen, z.B. Isobutylmethylketon, Estern, z.B. Essigsäurāthylester, aromatischen Lösungsmitten, z.B. Benzol ausführen, wobei es zweckmäßig ist, kräftig zu rühren, und den pH-Wert durch Basenzusatz oder Verwendung von Pufferlösungen in einem Bereich von etwa pH 2,0 bis 9,0, vorzugsweise zwischen 6,5 und 8,0, zu halten. Man kann die Reaktion aber auch in Wasser allein in Gegenwart einer organischen oder anorganischen Base oder unter Zusatz von Pufferstoffen durchführen.

Verwendet man als Ausgangsprodukte für das erfindungsgemäße Verfahren die Silylderivate der Verbindungen der allgemeinen Formel III (z.B. Mono- oder Di-trimethylsilylderivate) und setzt sie mit Verbindungen der allgemeinen

Formel IV um, so arbeitet man im allgemeinen zweckmäßigerweise in wasser- und hydroxyl-gruppenfreien Lösungsmitteln, beispielsweise in halogenierten Kohlenwasserstoffen, z.B. Methylenchlorid oder Chloroform, Benzol, Tetrahydrofuran, Aceton oder Dimethylformamid usw. Der Zusatz von Basen ist hierbei nicht notwendig, kann jedoch in einzelnen Fällen vorteilhaft sein, um die Ausbeute und Reinheit der Produkte zu verbessern. Als gegebenenfalls zugesetzte Basen werden zweckmäßigerweise tertiäre aliphatische oder aromatische Amine, wie Pyridin oder Triäthylamin, oder durch sterische Hinderung schweracylierbare sekundäre Amine, wie Dicyclohexylamin, benützt.

Statt der Silylester können auch alle anderen Carboxylderivate von $\alpha$-Aminobenzylpenicillinen, die auf dem Gebiet der Herstellung halbsynthetischer Penicilline bekannt sind, verwendet werden. Typische Beispiele sind die Tritylester, die p-Nitrobenzylester oder die Phenacylester. Im Anschluß an die Umsetzungen können diese Derivate nach bekannten Methoden in die erfindungsgemäßen Penicilline umgewandelt werden. Die Menge der verwendeten Basen ist z.B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der allgemeinen Formel II vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen und anorganischen Basen verwendet werden, wie Alkali- und Erdalkalihydroxide, Erdalkalioxide, Alkali- und Erdalkalicarbonate und Hydrogencarbonate, Ammoniak, primäre, sekundäre und tertiäre aliphatische und aromatische Amine sowie heterocyclische Basen. Beispielhaft seien Natrium-, Kalium- und Calziumhydroxid, Calziumoxid, Natrium- und Kaliumcarbonat, Natrium- und Kaliumhydrogencarbonat, Äthylamin, Methyl-äthylamin, Triäthylamin, Hydroxyäthylamin, Anilin, Pyridin und Piperidin genannt. Bei Verwendung der silylierten Ausgangsstoffe sollten jedoch die oben angegebenen Einschränkungen bezüglich der Art der Basen beachtet werden.

Als Puffersysteme können alle üblichen Puffermischungen verwendet werden, z.B. Phosphatpuffer, Citratpuffer und Tris-(hydroxymethyl)amino-methan-Puffer.

Die Reaktionstemperaturen können in einem größeren Bereich variiert werden. Im allgemeinen arbeitet man zwischen etwa -20 und etwa +50°C, vorzugsweise zwischen 0 und +20°C. Die Reaktionspartner der allgemeinen Formeln II und III können von vornherein in äquimolaren Mengen miteinander zur Reaktion gebracht werden. Es kann aber in einzelnen Fällen durchaus zweckmäßig sein, einen der beiden Reaktionspartner im Überschuß zu verwenden, um sich damit die Reinigung des Endproduktes zu erleichtern oder um die Ausbeute zu steigern.

2) Durch Umsetzung von Ureidocarbonsäuren der allgemeinen Formel V,

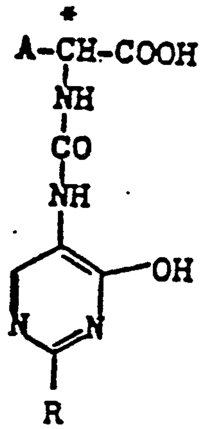

,(V)

in der A und R die oben angegebenen Bedeutungen besitzen, ihren Salzen oder reaktiven Derivaten, mit der 6-Aminopenicillansäure der Formel VI,

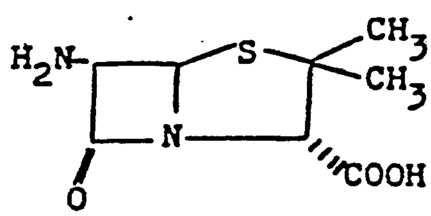

,(VI)

oder ihren anorganischen oder organischen Salzen oder Derivaten, die in die 6-Aminopenicillansäure leicht überführbar sind. Das dabei entstehende Reaktionsprodukt wird gegebenenfalls anschließend zu einem Penicillin der allgemeinen Formel I bzw. I' hydrolysiert oder katalytisch hydrogenolisiert.

Als reaktive Derivate der Ureidocarbonsäuren der allgemeinen Formel V kommen beispielsweise deren Säureanhydride wie z.B. die, die sich von Chlorameisensäureestern, z.B. Chlorameisensäureäthyl- oder -isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid oder deren Säureazide in Frage. Prinzipiell können jedoch alle Verknüpfungsmethoden, wie sie aus der ß-Lactamchemie bekannt sind, verwendet werden.

Die 6-Aminopenicillansäure wird vorteilhafterweise in Form eines ihrer Derivate eingesetzt. Als Derivate kommen hierfür z.B. in Frage: ihr Trimethylsilylester, Tritylester, p-Nitrobenzylester, Phenacylester und ihr O,N-Bis-trimethylsilylderivat. Diese Derivate werden bevorzugt in einem aprotischen Lösungsmittel, wie Methylenchlorid oder Tetrahydrofuran, umgesetzt. Man kann aber auch die 6-Aminopenicillansäure in Form ihrer Salze, beispielsweise ihres Triäthylammoniumsalzes, zum Einsatz bringen; hierbei benutzt man z.B. Methylenchlorid oder ein protisches Lösungsmittel oder ein wäßriges Medium oder ein wäßrig-organisches Lösungsmittel, wie z.B. Tetrahydrofuran-Wasser-Gemische.

Die Ureidocarbonsäure, ihre Salze oder ihre reaktiven Derivate werden mit der 6-Aminopenicillansäure oder ihren Derivaten in einem Lösungsmittel bei Temperaturen zwischen $-40^{o}C$ und $+40^{o}C$, gegebenenfalls in Gegenwart einer Base, umgesetzt. Wird z.B. ein Anhydrid der Ureidocarbonsäure, beispielsweise das Anhydrid mit dem Äthylchloroformiat, mit einem Derivat der 6-Aminopenicillansäure umgesetzt, so wird die Reaktion unter Kühlung, beispielsweise bei $-10^{o}C$ bis $+10^{o}C$ in einem Lösungsmittel, wie Aceton, Tetrahydrofuran, Dimethylformamid, Chloroform, Dichlormethan, Hexa-

metapol oder in einem Gemisch dieser Lösungsmittel, durchgeführt. Setzt man beispielsweise einen N-Hydroxysuccinimidester der Ureidocarbonsäure mit der 6-Aminopenicillansäure um, so wird die Reaktion vorzugsweise bei 0 bis 20°C in Anwesenheit einer Base, wie z.B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit der 6-Aminopenicillansäure oder mit deren Salzen erfolgt vorteilhafterweise in Gegenwart eines Kondensationsmittels, z.B. in Gegenwart von N,N'-Dicyclohexylcarbodiimid.

Wird ein Derivat der 6-Aminopenicillansäure eingesetzt, beispielsweise einer ihrer obengenannten Ester, so wird je nach den Reaktionsbedingungen gegebenenfalls ein Reaktionsprodukt erhalten, welches z.B. noch die Esterfunktion enthält. Ein solches Reaktionsprodukt ist aber leicht in das Penicillin der allgemeinen Formel I überführbar. Liegt zum Beispiel die Carboxylgruppe der 6-Aminopenicillansäure in Form ihres Silylesters vor, so kann sie nach der Reaktion in dem erhaltenen Penicillin der allgemeinen Formel I ebenfalls in Form ihres Silylesters vorliegen. In diesem Fall wird anschließend an die eigentliche Umsetzung diese Silylestergruppe abhydrolysiert, wodurch die Verbindung der allgemeinen Formel I bzw. I' entsteht. In anderen Fällen, z.B. bei Vorliegen eines p-Nitrobenzylesters, wird nach der eigentlichen Umsetzung diese p-Nitrobenzylestergruppe hydrogenolytisch gespalten, wobei dann das Penicillin der allgemeinen Formel I bzw. I' erhalten wird.

Die Aufarbeitung des nach beiden Verfahren erhaltenen Reaktionsgemisches nach erfolgter Umsetzung wird nach den bei ß-Lactam-Antibiotika gebräuchlichen Methoden vorgenommen; dasselbe gilt für die Isolierung und Reinigung der Endprodukte, beispielsweise für die Freisetzung der Säure aus ihren Salzen und die Überführung der freien Säure in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2-äthylhexanoat.

Die als Ausgangsstoffe verwendeten Verbindungen der allgemeinen Formel III sind literaturbekannt, vgl. z.B. E.H. Flynn, Cephalosporines and Penicillines, Academic Press, New York and London (1972).

Die Ausgangsstoffe der allgemeinen Formel IV können beispielsweise durch Umsetzung der entsprechenden 5-Aminopyrimidine der allgemeinen Formel VII,

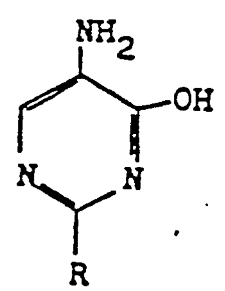

,(VII)

in der R wie oben definiert ist, mit Phosgen gewonnen werden. Diese Umsetzung erfolgt vorzugsweise in einem nicht Hydroxylgruppen-enthaltenden Lösungsmittel, wie Tetrahydrofuran, Methylenchlorid, Chloroform, Dimethoxyäthan oder Hexametapol bei Temperaturen zwischen -40° und +60°C, vorzugsweise zwischen -10° und +20°C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuß verwendet werden. Sollten die betreffenden Aminopyrimidine der

allgemeinen Formel VII in einem der erwähnten Lösungsmittel schwer löslich sein, kann die Phosgenierung auch in heterogener Phase durchgeführt werden. Desweiteren können die Aminopyrimidine der allgemeinen Formel VII durch Behandlung mit einem Silylierungsmittel, wie Hexamethyldisilazan, Trimethylchlorsilan/Triäthylamin oder Trimethylsilyldiäthylamin, in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel IV reagiert. Je nach der Art des Lösungsmittels, der Höhe der Temperatur, der Menge und Art der eingesetzten Base entsteht entweder überwiegend das entsprechende Isocyanat oder Carbaminsäurehalogenid oder ein Gemisch dieser beiden Verbindungen. Je nach den Reaktionsbedingungen kann die Verbindung der allgemeinen Formel IV auch geringoder teilweise als ein, den Isocyanaten isomeres Dihydro-oxazolo-pyrimidin der allgemeinen Formel IVa

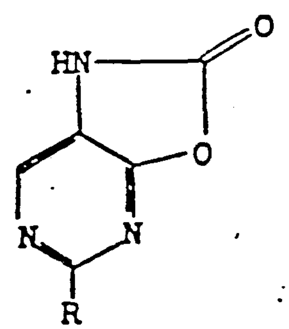

,(IVa)

vorliegen.

Die durch Phosgenierung entstandenen Ausgangsprodukte der allgemeinen Formel IV bzw. deren Gemische sind in den obenerwähnten Lösungsmitteln im allgemeinen gut löslich und können, nach Entfernung des überschüssigen Phosgens, ohne weitere Reinigung mit den entsprechenden Penicillinderivaten der allgemeinen Formel III direkt umgesetzt werden.

Die 5-Amino-4-hydroxy-2-subst.-Pyrimidine der allgemeinen Formel VII können z.B. nach folgenden Verfahren hergestellt werden:

Durch Umsetzung des 2-Chlor- oder des 2-Methylmercapto-4-hydroxy-5-nitropyrimidins der nachfolgenden Formel VIII (vgl. Vorbrüggen u. Strehlke, Chem. Ber. 106, S. 3039 (1973)) mit substituierten Anilinen der allgemeinen Formel IX oder IX', und anschließender Reduktion der Nitrogruppe der erhaltenen Verbindung der allgemeinen Formel X, bei der R wie eingangs definiert ist, nach bekannten Verfahren entsprechend folgendem Reaktionsschema:

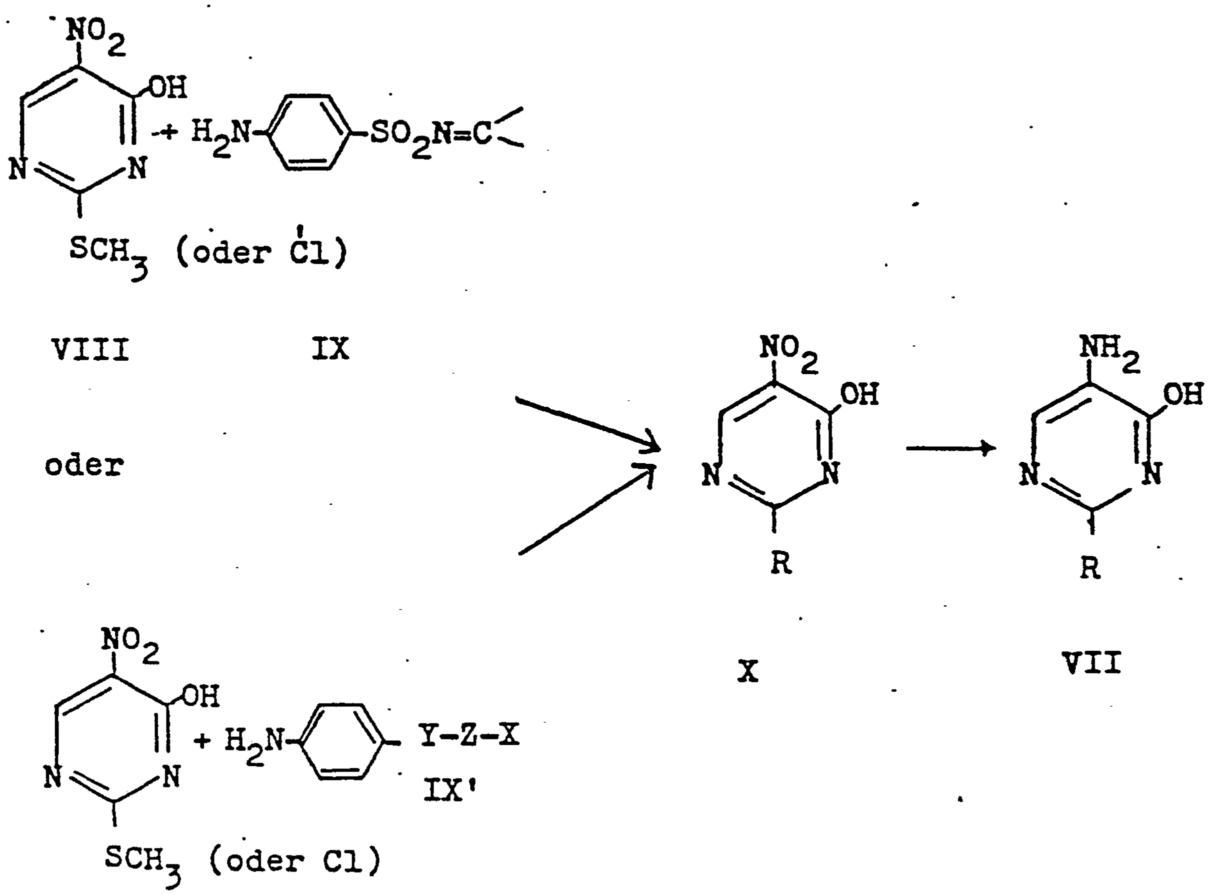

VIII IX

oder

X VII

IX'

Beispiele für die Darstellung der substituierten Aniline der allgemeinen Formel IX, sowie der Pyrimidine X bzw. VII werden im präparativen Teil gegeben.

Die Ureidocarbonsäuren der allgemeinen Formel V lassen sich durch Umsetzung der Pyrimidinderivate der allgemeinen Formel IV mit Glycinderivaten der allgemeinen Formel XI,

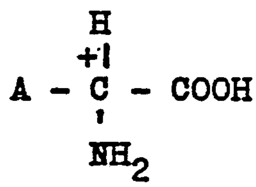
,(XI)

in der A wie oben definiert ist, erhalten. Die Umsetzung erfolgt bei Temperaturen zwischen -20°C und +40°C, vorzugsweise zwischen 0°C und +20°C, in einem Lösungsmittel. Als Lösungsmittel können hierbei z.B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin, oder anorganische Basen, wie verdünnte Natronlauge.

Es wurde gefunden, daß die Verbindungen der allgemeinen Formeln I bzw. I' wertvolle pharmakologische Eigenschaften bei guter Verträglichkeit besitzen. Die erfindungsgemäßen Wirkstoffe können daher zur Prophylaxe und Chemotherapie von lokalen und systemischen Infektionen in der Human- und Tiermedizin verwendet werden. Als Krankheiten, die durch die erfindungsgemäßen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen.

Dieses wird dadurch ermöglicht, daß die Verbindungen der allgemeinen Formeln I bzw. I' sowohl in vitro als auch in vivo gegen schädliche Mikroorganismen, sowohl gegen grampositive als auch besonders gegen gramnegative Bakterien und bakterienähnliche Mikroorganismen sehr stark wirken, wobei sie sich durch ein breites Wirkungsspektrum auszeichnen.

Mit diesen Cephalosporinderivaten können beispielsweise lokale und/oder systemische Erkrankungen behandelt und/oder verhindert werden, die durch die folgenden Erreger oder durch Mischungen der folgenden Erreger verursacht werden:
Micrococcaceae, wie Staphylokokken;
Lactobacteriaceae, wie Streptokokken;
Neisseriaceae, wie Neisserien;
Corynebacteriaceae, wie Corynebakterien;
Enterobacteriaceae, wie Escherichiae-Bakterien der Coli-Gruppe;
Klebsiella-Bakterien, z.B. K. pneumoniae;
Proteae-Bakterien der Proteus-Gruppe z.B. Proteus vulgaris;
Salmonella-Bakterien, z.B. S.thyphimurium;
Shigella-Bakterien, z.B. Shigella dysenteriae;
Pseudomonas-Bakterien, z.B. Pseudomonas aeruginosa;
Aeromonas-Bakterien, z.B. Aeromonas lique faciens;
Spirillaceae, wie Vibrio-Bakterien, z.B. Vibrio cholerae;
Parvobacteriaceae oder Brucellaceae, wie Pasteurella-Bakterien;
Brucella-Bakterien, z.B. Brucella abortus;
Haemophilus-Bakterien, z.B. Haemophilus influenzae;
Bordetella-Bakterien, z.B. Bordetella pertussis;
Moraxella-Bakterien, z.B. Moraxella lacunata;
Bacteroidaceae, wie Bacteroides-Bakterien;
Fusiforme-Bakterien, z.B. Fusobacterium fusiforme;
Sphaerophorus-Bakterien, z.B. Sphaerophorus necrophorus;
Bacillaceae, wie aerobe Sporenbildner, z.B. Bacillus anthracis;
anaerobe Sporenbildner-Chlostridien, z.B. Chlostridium perfringens;
Spirochaetaceae, wie Borrelia-Bakterien;
Treponema-Bakterien, z.B. Treponema pallidum;
Leptospira-Bakterien, wie Leptospira interrogans.

Die obige Aufzählung von Erregern ist lediglich beispielhaft und keineswegs beschränkend aufzufassen.

In folgenden werden einige typische besonders gut wirksame Penicilline gemäß vorliegender Erfindung aufgezählt:

D-α-[3-(4-Hydroxy-2-p-(3',4',5',6'-tetrahydro-pyrimidin-2'-yl)-aminosulfonylanilino -5-pyrimidinyl)-ureido]-p-hydroxybenzylpenicillin-Natrium,
D-α-[3-(4-Hydroxy-2-p-(4',5'-dihydro-oxazol-2'-yl)-aminosulfonylanilino-5-pyridmidinyl)-ureido]-p-hydroxybenzylpenicillin-Natrium,
D-α-[3-(4-Hydroxy-2-p-(4',5'-dihydro-thiazol-2'-yl)-aminosulfonylanilino-5-pyrimidinyl)-ureido]-p-hydroxybenzylpenicillin-Natrium,
D-α-[3-(4-Hydroxy-2-p-(3',4',5',6'-tetrahydro-pyrimidin-2'-yl)-aminosulfonylanilino -5-pyrimidinyl)-ureido]-benzylpenicillin-Natrium,
D-α-[3-(4-Hydroxy-2-p -(4',5'-dihydro-imidazolyl-2'-yl)-aminosulfonylanilino-5-pyrimidinyl)-ureido]-p-hydroxybenzylpenicillin-Natrium,
D-α-[3-(4-Hydroxy-2-p-(1'-methyl-3',4',5',6'-tetrahydro-pyrimidin-2'-yl)-aminosulfonylanilino -5-pyrimidinyl)-ureido]-p-hydroxybenzylpenicillin-Natrium,
D-α-[3-(4-Hydroxy-2-p -(4'-methyl-4',5'-dihydro-imidazolyl-2'-yl)-aminosulfonylanilino-5-pyrimidinyl)-ureido]-p-hydroxybenzylpenicillin-Natrium,
D-α-[3-(4-Hydroxy-2-p-(amino-methylthio)-methylen-iminosulfonylanilino -5-pyrimidinyl)-ureido]-p-hydroxybenzylpenicillin-Natrium,
D-α-[3-(4-Hydroxy-2-p-(2'-hydroxyäthyl)-sulfinylanilino-5-pyrimidinyl)-ureido]-p-hydroxybenzylpenicillin-Natrium,
D-α-[3-(4-Hydroxy-2-p-(2'-hydroxyäthyl)-sulfinylanilino-5-pyrimidinyl)-ureido]-benzylpenicillin-Natrium,
D-α-[3-(4-Hydroxy-2-p-(2'-hydroxyisopropyl)-sulfinylanilino-5-pyrimidinyl)-ureido]-p-hydroxybenzylpenicillin-Natrium,

D-α-/3-(4-Hydroxy-2-p-(2'-hydroxyäthylamino)-sulfonyl-anilino-5-pyrimidinyl)-ureido7-p-hydroxybenzylpenicillin-Natrium,
D-α-/3-(4-Hydroxy-2-p-(2'-acetylaminoäthylamino)-sulfonyl-anilino-5-pyrimidinyl)-ureido7-p-hydroxybenzylpenicillin-Natrium,
D-α-/3-(4-Hydroxy-2-p-(aminocarbonylmethylamino)-sulfonyl-anilino-5-pyrimidinyl)-ureido7-p-hydroxybenzylpenicillin-Natrium,
D-α-/3-(4-Hydroxy-2-p-(3'-aminocarbonylpropylamino)-sulfonyl-anilino-5-pyrimidinyl)-ureido7-p-hydroxybenzylpenicillin-Natrium.

Die Wirksamkeit der erfindungsgemäßen ß-Lactam-Antibiotika läßt sich durch folgende Untersuchungen beispielhaft demonstrieren:

1.) <u>In vitro-Versuche</u>

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht:
128; 64; 32; 16; 8; 4; 2; 1; 0,5; 0,25; 0,12; 0,06 µg/ml.
Es wurde ein Nährboden folgender Zusammensetzung benutzt: 10 g Pepton, 8 g Fleischextrakt-Oxoid, 3 g Natriumchlorid, 2 g sek. Natriumphosphat werden mit dest. Wasser auf 100 ml aufgefüllt (pH 7,2-7,4). Das Alter der Primärkulturen betrug etwa 20 Stunden. Die Einstellung der Keimsuspension erfolgte am Photometer (nach "Eppendorf") (Reagenzglas-Durchmesser 14 mm, Filter 546 nm) anhand der Trübung einer Bariumsulfat-Vergleichssuspension, die durch eine Bariumsulfat-Aufschwemmung erzeugt wurde, welche durch die Zugabe von 3,0 ml 1 %ige Barium-chloridlösung in 97 ml 1 %ige Schwefelsäure entstand. Nach der Einstellung wurden Streptococcus Aronson im Verhältnis 1:15 und die übrigen Testkeime im Verhältnis 1:1500 mit einer

weiter verdünnt.
16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37°C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benützt:

Staphylococcus aureus SG 511, Escherichia coli ATCC 11 775, Pseudomonas aeruginosa Hamburgensis und Pseudomonas aeruginosa BC 19, Serratia marcescens ATCC 13 880, Klebsiella pneumoniae ATCC 10 031 und BC 6, Proteus mirabilis Hamburgensis, Proteus rettgeri BC 7, Enterobacter cloacae ATCC 13 047.

In der folgenden Tabelle 1 sind die ermittelten minimalen Hemmkonzentrationen (MIC) für typische Vertreter der erfindungsgemäßen Verbindungen aufgeführt. Es handelt sich um die Natriumsalze von Verbindungen der allgemeinen Formel I mit A=p-Hydroxyphenyl und folgender Bedeutung von R:

| R | Verbindung |
|---|---|
| 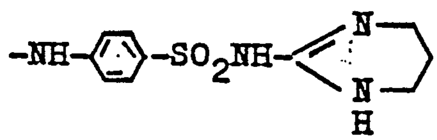 | A |
| 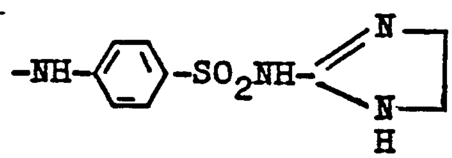 | B |
| 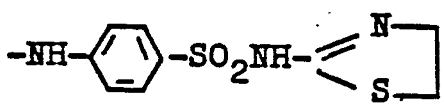 | C |
| 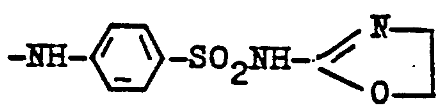 | D |
| 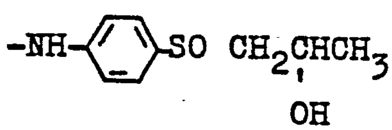 | E |
| 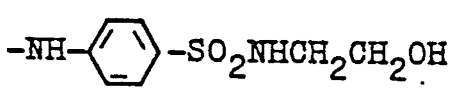 | F |
| 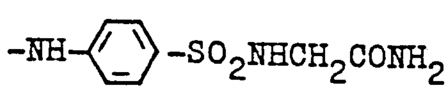 | G |

| R | Verbindung |
|---|---|
| 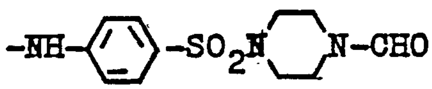 | H |
| und im Vergleich hierzu um das | |
| Azlocillin | J |

Tabelle 1

| Substanz | S.aureus SG 511 | E.Coli ATCC 11775 | Pseud. aerug. Hbg. | Pseud. aerug. BC 19 | Serrat. marcesc. ATCC 13880 | Klebs. pneum. ATCC 10031 | Klebs. pneum. BC 6 | Prot. mira-bilis Hbg | Prot. rett-geri BC 7 | Eb. cloacae ATCC 13047 |
|---|---|---|---|---|---|---|---|---|---|---|
| A | 0,25 | 0,25 | 1 | 1 | 0,5 | 2 | 2 | 0,06 | 0,5 | 0,5 |
| B | 0,5 | 0,25 | 2 | 1 | 0,25 | 2 | 4 | 0,06 | 0,5 | 0,1 |
| C | 0,25 | 0,12 | 1 | 2 | 0,25 | 1 | 1 | 0,06 | 0,25 | 0,5 |
| D | 0,5 | 0,25 | 1 | 1 | 0,25 | 2 | 4 | 0,06 | 0,25 | 1 |
| E | 0,5 | 0,25 | 1 | 1 | 0,5 | 2 | 2 | 0,03 | 0,5 | 1 |
| F | 0,5 | 0,25 | 2 | 1 | 0,13 | 2 | 4 | 0,06 | 0,5 | 2 |
| G | 0,5 | 0,12 | 1 | 1 | 0,25 | 2 | 2 | 0,06 | 0,5 | 1 |
| H | 0,5 | 0,25 | 2 | 1 | 0,25 | 2 | 2 | 0,06 | 0,5 | 1 |
| J | 0,5 | 8 | 8 | 8 | 4 | 32 | 32 | 2 | 8 | 32 |

Die akute Toxizität wurde durch perorale und subcutane Applikation der Verbindungen der Tabelle 1 und 2 an weißen Laboratoriumsmäusen in steigenden Dosen bestimmt.

Die $LD_{50}$ ist die Dosis, nach deren Applikation 50 % der Tiere innerhalb von 8 Tagen sterben. Sämtliche Substanzen zeigten bei oraler Gabe eine $LD_{50}$ von über 4 g/kg, bei subcutaner Gabe eine $LD_{50}$ von über 2 g/kg, d.h. bei 2 g/kg starben keine Tiere, sie sind damit für die Praxis untoxisch.

Eine Reihe der erfindungsgemäßen Verbindungen wurde in vivo bei experimentellen Infektionen an Mäusen untersucht. Man verwendete als pathogene Bakterien E.coli ATCC 11775 und Pseudomonas aeruginosa BC 19. Es wurde eine intraperitoneale Infektion mit 0,2 ml einer Bakteriensuspension (mit 5 % Mucin) gesetzt. Dies entspricht etwa $1,4x10^6$ Keime E.coli bzw. $1,3x10^6$ Keime Pseudomonas/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemäßen Cephalosporine subcutan zur Bestimmung der $ED_{50}$ (Dosis bei der 50 % der Tiere überleben) behandelt. Bei der E. coli-Infektion wurde einmal therapiert (1 Stunde post-Infektionem). Bei der Pseudomonas-Infektion wurde 3x (1,3,5 Stunden post-Infektionem) behandelt.

Der Beobachtungszeitraum betrug in beiden Fällen 7 Tage. Die Ergebnisse dieser Teste mit repräsentativen Vertretern der erfindungsgemäßen Penicilline sind in der Tabelle 2 dargestellt.

Tabelle 2

| Verbindung | E. coli - Infektion $ED_{50}$ (mg/kg) | Verbindung | Pseud. Infektion $ED_{50}$ (mg/kg) |
|---|---|---|---|
| A | 0,6 | A | 2,1 |
| B | 0,7 | B | 1,9 |
| C | ~ 3 | C | 17 |
| D | ~ 2 | E | ~10 - 20 |
| E | ~ 2 | F | ~10 - 20 |
| F | ~5 | | |
| G | ~ 5 | | |
| J | > 100 | J | > 100 |

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragées, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einer Dosierung zwischen 5 und 500, vorzugsweise 10-200 mg/kg Körpergewicht je 24 Stunden verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitpunkt bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Beispiele zur Herstellung der Ausgangsprodukte:

Beispiel A

5-Amino-4-hydroxy-2-{p-(3',4',5',6'-tetrahydro-pyrimidin-2'-yl)-aminosulfonylanilino}-pyrimidin

27,6 g (0,1 Mol) 4-(Bismethylthio-methylen-imino-)sulfonyl-anilin (C.A. 63, 8343b (1965)) und 9,2 g (0,1 Mol) 1,3 Diaminopropan werden in 200 ml wasserfreiem Äthanol 3 Stunden zum Sieden erhitzt. Der entstandene Niederschlag wird heiß abgesaugt. Man erhält 17,5 g (69 % der Theorie) p-(3',4',5',6'-tetrahydro-pyrimidin-2'-yl)-aminosulfonyl-anilin vom F.p. 223-225°C.

3,4 g (13,4 mmol) dieser Verbindung werden in 80 ml Wasser auf 90°C erwärmt und 3,5 g (17,7 mmol) 2-Chlor-4-hydroxy-5-nitro-pyrimidin-Natrium portionsweise zugegeben. Nach 1 Stunde Rühren bei 90°C wird gekühlt und abgesaugt. Man erhält 3,2 g (60 % der Theorie) 2-{p-(3',4',5',6'-Tetrahydro-pyrimidin-2'-yl)-aminosulfonyl-anilino}-4-hydroxy-5-nitro-pyrimidin vom F.p. > 270°C.

4 g (11 mmol) dieses Produkts werden in einem Gemisch von 50 ml konz. wässrigem Ammoniak und 100 ml Wasser gelöst. Nach Zugabe von 8,9 g (51 mmol) Natriumdithionit wird eine Stunde bei Raumtemperatur gerührt. Unter Eiskühlung wird dann Essigsäure bis zum pH-Wert von 6,9 bis 7,0 zugegeben, das ausgefallene Produkt mit wenig Eiswasser gewaschen und getrocknet. Man erhält 3,1 g (85 % der Theorie) der Titelverbindung, die sich ab 250°C zersetzt.

Das Produkt fällt gelegentlich nicht kristallin, sondern ölig an. In diesem Fall wird vom Öl abdekantiert, mit Eiswasser gewaschen, mit Methanol verrührt, abgesaugt und mit Äther gewaschen.
NMR-Spektrum (DMSO + $CD_3OD$) Signale bei ppm (80 MHz):
1,8(m,2H), 3,25(m,4H), 7,2(s,1H), 7,7(s,4H).

Auf diese Weise wurden die in der folgenden Tabelle dargestellten Verbindungen der allgemeinen Formel VII erhalten:

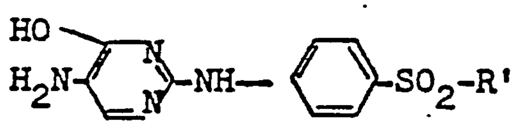

| Beispiel | R' | NMR-Spektrum (DMSO + $CD_3OD$) Signale bei ppm (80 MHz) |
|---|---|---|
| a) | 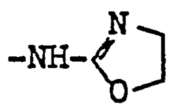 | 3,6(m,2H), 4,4(m,2H), 7,1(s,1H), 7,65 (s,4H). |
| b) | 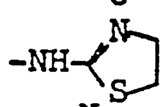 | 3,3-3,8(m,4H), 7,25(s,1H), 7,8(s,4H). |
| c) | 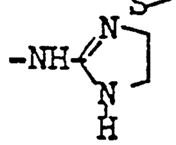 | 3,6(t,4H), 7,1(s,1H), 7,65(s,4H). |
| d) | 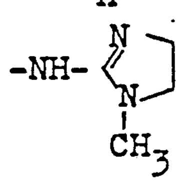 | 2,8(s,3H), 3,5(s,4H), 7,25(s,1H), 7,7(s,4H). |
| e) | 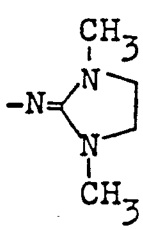 | 2,95(s,6H), 3,6(s,4H), 7,2(s,1H), 7,7(s,4H). |

| Beispiel | R' | NMR-Spektrum (DMSO + $CD_3OD$) Signale bei ppm (80 MHz) |
|---|---|---|
| f) | 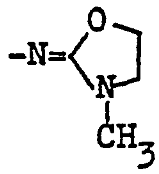 | 2,9(s,3H), 3,7(m,2H), 4,5(m,2H), 7,2(s,1H), 7,7(s,4H). |
| g) | 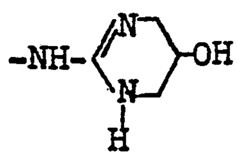 | 3,2(m,4H), 7,2(s,1H), 7,65(s,4H). |
| h) | 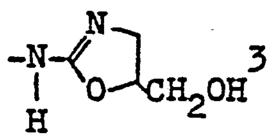 | 3,6(m,4H), 4,7(m,1H), 7,2(s,1H), 7,75(s,4H). |
| i) | 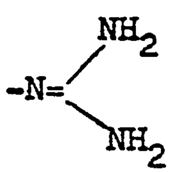 | 7,1(s,1H), 7,65(s,4H). |
| j) | 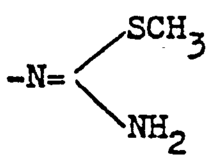 | 2,25(s,3H), 7,1(s,1H), 7,7(s,4H). |
| k) | 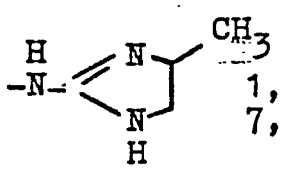 | 1,2(d,3H), 3,1(m,1H), 3,5-3,9(m,2H), 7,15(s,1H), 7,7(s,4H). |
| l) | 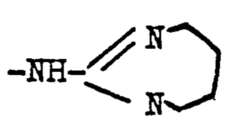 | 1,5(s breit, 4H), 3,1(s breit, 4H), 7,15(s,1H), 7,65(s,4H) |

| Beispiel | R' | NMR-Spektrum (DMSO+$CD_3OD$) Signale bei ppm (80 MHz) |
|---|---|---|
| m | 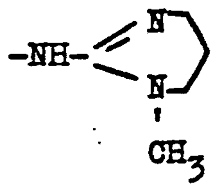 | 1,8(m,2H), 2,9(s,3H), 3,2(m,4H), 7,15(s,1H), 7,65(s,4H). |
| n | 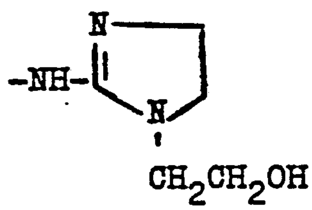 | 3,1-3,5(m+s breit,8H), 7,1(s,1H), 7,6(s,4H). |

## Beispiel B

**5-Amino-4-hydroxy-2-p-(2'-hydroxyäthyl)-aminosulfonyl-anilino-pyrimidin**

22,1 g p-Nitrobenzolsulfonsäurechlorid werden in 100 ml Aceton gelöst und bei Eiskühlung mit einer Lösung von 12,5 g Aminoäthanol in Wasser versetzt. Man läßt zwei Stunden bei Raumtemperatur stehen und saugt dann das ausgefallene Festprodukt ab.
Ausbeute: 17,9 g (73 %) p-Nitrobenzol-(2'-hydroxyäthyl)-sulfonamid.

Dieses Produkt wird in 300 ml Methanol aufgeschlämmt und mit 4 g Raney-Nickel bei Raumtemperatur und Normaldruck hydriert. Bei der Hydrierung tritt Lösung ein.
Man erhält 14,4 g p-(2'-Hydroxyäthyl)-aminosulfonylanilin.

9,5 g 4-Hydroxy-2-methylmercapto-5-nitro-pyrimidin und 12 g des oben erhaltenen Anilino-derivats werden in 100 ml Eisessig unter Rühren zum Rückfluß erwärmt, wobei der Eisessig abdestilliert wird. Die zurückbleibende Schmelze wird 15 Minuten lang auf 170°C erwärmt. Nach dem Abkühlen wird das zurückbleibende Festprodukt in Äthanol aufgeschlämmt, ausgerührt und abgesaugt.
Ausbeute: 11,1 g (62 %) 4-Hydroxy-2-(2'-hydroxyäthyl)-p-sulfonylanilino-5-nitro-pyrimidin.

Alternativ kann dieses Nitropyrimidin auch wie folgt hergestellt werden:
Man rührt 4,3 g 2-Chlor-4-hydroxy-5-nitropyrimidin-Monohydrat-Natriumsalz mit 4,4 g p-(2'-Hydroxyäthyl)-aminosulfonylanilin eine Stunde lang in 100 ml Wasser bei 80°C. Das ausgefallene Nitropyrimidin wird abgesaugt und getrocknet.

8 g der nach diesen Verfahren erhaltenen Nitroverbindung werden zusammen mit 3 g Palladium auf Kohle als Katalysator (20 %ig) in 150 ml Methanol, dem man 15 ml konz. Salzsäure hinzufügt, aufgeschlämmt. Es wird unter Schütteln bei Raumtemperatur hydriert.
Die Wasserstoffaufnahme (~1,5 l) ist nach etwa 50 Minuten beendet. Man filtriert vom Katalysator ab und dampft zur Trockene ein. Das zurückbleibende farblose Hydrochlorid wird in 100 ml Wasser gelöst und mit konz. Natronlauge unter Eiskühlung versetzt bis ein pH von 4,5 erreicht ist. Das oxidationsempfindliche, ausgefallene Produkt wird rasch abgesaugt und getrocknet. Zur Entfernung von Verunreinigungen wird in sehr wenig Dimethylformamid gelöst und rasch über eine Silicagelsäule (Eluens: Methylenchlorid/Methanol 3:1) chromatographiert.
Ausbeute: 3,8 g (52 %) farbloses Aminopyrimidin.

Diese Reduktion kann auch wie folgt durchgeführt werden:
Man löst 3,55 g Nitropyrimidin zusammen mit 10 ml konz. Ammoniak unter leichtem Erwärmen in 40 ml Wasser und gibt unter Rühren 8 g Natriumdithionit zur Lösung. Es tritt eine Entfärbung der zunächst gelben Lösung ein. Zur Vervollständigung der Reaktion wird 15 Minuten auf etwa 60°C erwärmt. Anschließend wird abgekühlt und unter Eiskühlung mit konz. Salzsäure bis pH 5,0 versetzt. Das ausgefallene Produkt wird abgesaugt und getrocknet. Unter Umständen kann entsprechend dem vorhergehenden Beispiel mit Hilfe einer Silicagelsäule gereinigt werden.

Ausbeute an Rohprodukt: 1,85 g;
NMR-Spektrum (DMSO + $CD_3OD$) Signale bei ppm: 3,2 (m,2H), 3,65(m,2H), 7,2(s,1H), 7,8(s,4H).

Nach diesen Methoden wurden die Aminopyrimidine der folgenden Tabelle synthetisiert.

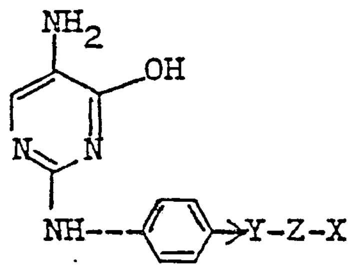

| | → Y-Z-X | NMR-Spektrum (DMSO + $CD_3OD$) Signale bei ppm: |
|---|---|---|
| a) | $-SO_2N(CH_2CH_2OH)_2$ | 3,25(m,4H), 3,70(m,4H), 7,15 (s,1H), 7,8(s,4H). |
| b) | 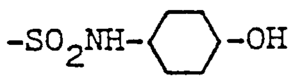 | 1,2-1,8(m,8H), 3,0(m,1H), 3,6 (m,1H), 7,2(s,1H), 7,75(s,4H). |
| c) | 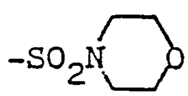 | 2,85(breites t,4H), 3,6(breites Signal,4H), 7,20(s,1H), 7,75(q,4H). |
| d) | $-SO_2NHCH_2CONH_2$ | 3,55(t,2H), 7,3(s,1H), 7,7(s,4H). |
| e) | 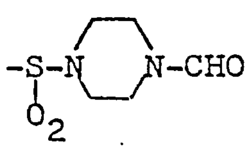 | 2,9(m,4H), 3,45(m,4H), 7,25(s,1H), 7,75(q,4H), 7,95(s,1H). |
| f) | $-SO_2NH(CH_2)_3CONH_2$ | 1,70(q,2H), 2,15(t,2H), 2,75 (t,2H), 7,20(s,1H), 7,8(breites s, 4H). |
| g) | $-SO_2NHCH_2CH_2NHCOCH_3$ | 1,8(s,3H), 2,8(m,2H), 3,2(m,2H), 7,2(s,1H), 7,7(s,4H). |
| h) | $-SO_2NHCH_2CH_2COOH$ | 2,4(t,2H), 2,95(t,2H), 7,2(s,1H), 7,75(q,4H). |

Beispiel C

5-Amino-4-hydroxy-2-p-(2'-hydroxyäthyl)-sulfinylanilino-pyrimidin

Zur Lösung von 1,2 g Natrium (0,052 Mol) in 50 ml Äthanol werden 4,1 g (0,052 Mol) 2-Mercaptoäthanol zugetropft. Zur Lösung des Mercaptids werden unter Eiskühlung 7,05 g (0,05 Mol) 4-Fluornitrobenzol zugetropft. Der Ansatz wird 10 Minuten unter Rückfluß erwärmt und im Vakuum eingeengt. Der Rückstand wird in Wasser aufgenommen und das abgeschiedene Öl mit Essigester ausgeschüttelt. Nach dem Trocknen und Abdestillieren des Essigesters erhält man ein Öl, das bei längerem Stehen kristallisiert.
Ausbeute: 9,1 g (91,4 %) 2-Hydroxyäthyl-p-nitrophenyl-sulfid.

7,1 g (0,035 Mol) 2-Hydroxyäthyl-p-nitrophenyl-sulfid werden in 50 ml Eisessig gelöst, 4 ml 30%iges Wasserstoffperoxid (0,038 Mol) zugefügt und 3 Tage im Kühlschrank stehengelassen. Nach dem Abdestillieren der Lösungsmittel im Vakuum wurde der Rückstand durch Chromatographie über eine Kieselgelsäule Fließmittel Chloroform/Methanol 30:1) gereinigt.
Ausbeute: 3,8 g (48 %) 2-Hydroxyäthyl-p-nitrophenyl-sulfoxid.

Eine Lösung von 3,7 g (0,017 Mol) 2-Hydroxyäthyl-p-nitrophenyl-sulfoxid werden in 200 ml Methanol gelöst und in Gegenwart von 0,5 g Raney-Nickel bei Raumtemperatur und 5 bar hydriert. Nach dem Abfiltrieren des Katalysators wird zur Trockene eingedampft.
Ausbeute: 3,2 g (100 %) p-(2-Hydroxyäthyl)-sulfinyl-anilin.

Zur Lösung von 3,2 g (0,017 Mol) p-(2-Hydroxyäthyl)-sulfinyl-anilin werden 3,7 g (0,017 Mol) 2-Chlor-4-hydroxy-5-nitro-pyrimidin-Natrium mal 1$H_2O$ zugelöst.

Die Lösung wird 30 Minuten auf dem Dampfbad erhitzt, abgekühlt und der gebildete Niederschlag abgesaugt.
Schmelzpunkt: 256°C (Zers.).
Ausbeute: 3,7 g (65 %) 4-Hydroxy-2-p-(2'-hydroxyäthyl)-sulfinyl-anilino-5-nitropyrimidin.

1 g (0,003 Mol) 4-Hydroxy-2-p-(2'-hydroxyäthyl)-sulfinyl-anilino-5-nitropyrimidin wird in 10 ml konzentriertem Ammoniak und 40 ml Wasser gelöst, 2,7 g (0,015 Mol) Natriumdithionit zugesetzt und 30 Minuten auf dem Dampfbad erhitzt. Die Lösung wird zur Trockene eingedampft und der Rückstand mit Methanol ausgekocht. Das Rohprodukt wird durch Chromatographie über eine Kieselgelsäule (Fließmittel Chloroform/Methanol 2:1) gereinigt.
Ausbeute: 290 mg (32 %) 5-Amino-4-hydroxy-2-p-(2'-hydroxyäthyl)-sulfinyl-anilino-pyrimidin.
NMR-Spektrum (DMSO + $CD_3OD$) Signale bei ppm:
2,9(t,2H), 3,7(m,2H), 7,15(s,1H), 7,65(q,4H).

Nach dieser Methode wurden die Aminopyrimidine der folgenden Tabelle synthetisiert:

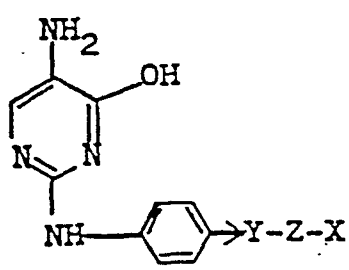

| | →Y-Z-X | NMR-Spektrum (DMSO + $CD_3OD$), Signale bei ppm: |
|---|---|---|
| a) | $-SO_2-CH_2CH_2-OH$ | 3,5(d,d,4H), 7,1(s,1H), 7,7(s,4H). |
| b) | $-SO_2-CH_2CH_2-SO_2-CH_3$ | 3,05(s,3H), 3,5(m,4H), 7,2(s,1H), 7,8(s,4H). |
| c) | 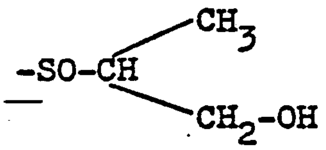 | 0,9(t,3H), 2,8(m,1H), 3,65(m,2H), 7,15(s,1H), 7,7(m,4H). |
| d) | 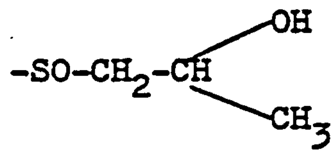 | 1,25(m,3H), 2,9(m,2H), 3,8(m,1H), 7,25(s,1H), 7,75(m,4H). |
| e) | 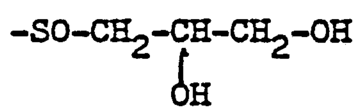 | 2,9(m,2H), 3,45(d,2H), 3,85(m,1H), 7,2(s,1H), 7,75(q,4H). |

## Beispiel D

D,L-α-/3-(2-p-(2'-Hydroxyäthyl)-aminosulfonyl-anilino)-5-pyrimidinyl)-ureido/-thienylglycin

1,63 g (5 mMol) 5-Amino-4-hydroxy-2-p-(2'-hydroxyäthyl)-aminosulfonylanilino-pyrimidin werden in 50 ml wasserfreiem Tetrahydrofuran suspendiert und mit 20 ml Trimethylsilyldiäthylamin bis zur Lösung gerührt (ca. 1 Stunde bei 20 bis 40°C). Von eventuell verbleibenden unlöslichen Anteilen wird unter Feuchtigkeitsausschluß filtriert und das Filtrat zur Trockne eingedampft. Der Rückstand wird in 50 ml Tetrahydrofuran aufgenommen und zu einer eisgekühlten Lösung von 0,5 g (5 mmol) Phosgen in 20 ml wasserfreiem Tetrahydrofuran getropft. Das Gemisch wird 15 Minuten bei Raumtemperatur gerührt, dann zur Trockne eingedampft und in 50 ml Tetrahydrofuran aufgenommen (Lösung I).

800 mg (5,5 mmol) D,L-α-Thienylglycin werden in 100 ml 80%igem wäßrigen Tetrahydrofuran suspendiert und mit 5,5 ml 1N-Natronlauge versetzt, wobei Lösung eintritt. Bei 0 bis 5°C wird Lösung I zugetropft und anschließend 30 Minuten bei 0°C und eine Stunde bei Raumtemperatur gerührt. Dann werden 100 ml Wasser zugegeben und das Tetrahydrofuran im Vakuum entfernt. Die wäßrige Phase wird mit Essigester extrahiert, die organische Phase verworfen und die wäßrige Phase mit 2 N-Salzsäure auf pH 2,6 gebracht. Der Niederschlag wird abgesaugt und getrocknet. Man erhält 1,45 g (57 %) der Titelverbindung.

NMR-Spektrum (DMSO + $CD_3OD$) Signale bei ppm:
3,2(m,2H), 3,60(m,2H), 5,50(s,1H), 7,0(m,2H), 7,4(d,1H), 7,8(s,4H), 8,34(s,1H).

Analog wurden die Ureidocarbonsäuren der folgenden Tabelle hergestellt:

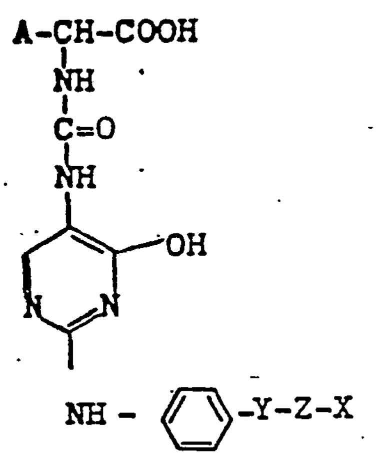

| | A | —Y-Z-X | NMR-Spektrum (DMSO + $CD_3OD$) Signale bei ppm: | Ausbeute % |
|---|---|---|---|---|
| a) | 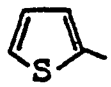 | $-SO_2NH(CH_2)_3CONH_2$ | 1,75(m,2H), 2,20(m,2H), 2,85(m,2H), 5,5(s,1H), 7,0(m,2H), 7,45(d,1H), 7,75(s,4H), 8,35(s,1H). | 46 |
| b) | 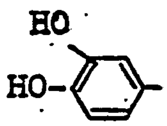 | $-SO_2NHCH_2CH_2OH$ | 3,15(m,2H), 3,55(m,2H), 5,20(s,1H), 6,7(s,2H), 6,85(s,1H), 7,75 (breites s,4H), 8,30 (s,1H). | 52,5 |
| c) | 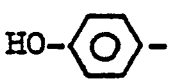 | 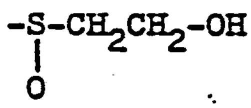 | 2,9(m,2H), 3,75(m,2H), 5,15(s,1H), 6,80(d,2H), 7,25(d,2H), 7,70(q,4H), 8,32(s,1H). | 42 |

Beispiel E

D-α-/3-(4-Hydroxy-2-{p-(3',4',5',6'-tetrahydro-pyrimidin-2'-yl)-aminosulfonyl-anilino}-5-pyrimidinyl)-ureido7-p-hydroxy-phenylglycin

2,0 g (5,5 mmol) 5-Amino-4-hydroxy-2-{p-(3',4',5',6'-tetrahydro-pyrimidin-2'-yl)-aminosulfonyl-anilino}-pyrimidin werden in 50 ml wasserfreiem Tetrahydrofuran suspendiert und mit 20 ml Trimethylsilyldiäthylamin bis zur Lösung gerührt (ca. 1 Stunde bei 20-40°C). Von eventuell verbleibenden unlöslichen Anteilen wird unter Feuchtigkeitsausschluß filtriert und das Filtrat zur Trockne eingedampft. Der Rückstand wird in 50 ml Tetrahydrofuran aufgenommen und zu einer eisgekühlten Lösung von 0,544 g (5 mmol) Phosgen in 20 ml wasserfreiem Tetrahydrofuran getropft. Das Gemisch wird 15 Minuten bei Raumtemperatur gerührt, dann zur Trockne eingedampft und in 50 ml Tetrahydrofuran aufgenommen (Lösung I).

0,6 g (3,6 mmol) D-α-p-Hydroxyphenylglycin werden in 100 ml 80%igem wässrigem Tetrahydrofuran suspendiert und mit 3,6 ml 1 N-Natronlauge versetzt, wobei Lösung eintritt. Bei 0-5°C wird Lösung I zugetropft und anschließend 30 Minuten bei 0°C und eine Stunde bei Raumtemperatur gerührt. Dann werden 100 ml Wasser zugegeben und das Tetrahydrofuran im Vakuum entfernt. Die wässrige Phase wird mit Essigester extrahiert, die organische Phase verworfen und die wässrige Phase mit 2N-Salzsäure auf pH 2,6 gebracht. Der Niederschlag wird abgesaugt und getrocknet. Man erhält 0,9 g (45 %)der Titelverbindung, die sich bei etwa 250°C zersetzt.
NMR-Spektrum (DMSO + $CD_3OD$) Signale bei ppm (80 MHz):
1,75 (m,2H), 3,2(m,4H), 5,15(s,1H), 6,8-7,25(q,4H), 7,75 (s,4H), 8,3(s,breit,1H).

Auf analoge Weise wurden die in der folgenden Tabelle zusammengefaßten Verbindungen der allgemeinen Formel V erhalten:

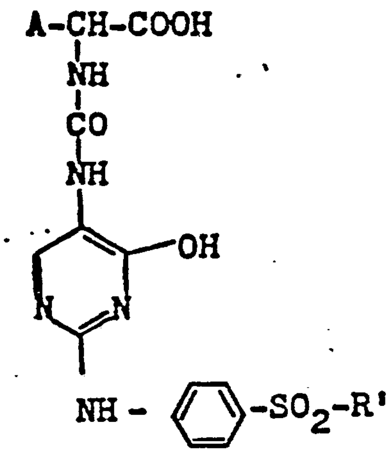

| Beispiel | A | R' | NMR-Spektrum (DMSO + $CD_3OD$) Signale bei ppm (80 MHz) |
|---|---|---|---|
| a) | 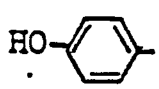 | 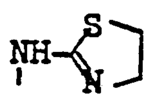 | 3,3-3,7(m,4H), 5,15(s,1H), 6,8/7,25(q,4H), 7,75(s,4H), 8,3(s,1H). |
| b) | 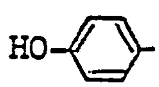 | 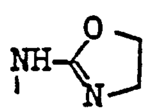 | 3,15(m,2H), 3,4(q,2H), 5,1 (s,1H), 6,75/7,2(q,4H), 7,7 (s,4H), 8,25(s,breit,1H). |
| c) | 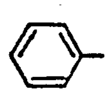 | 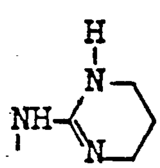 | 1,75(m,2H), 3,25(m,4H), 5,10 (s,1H), 7,40-7,80(m,9H), 8,25(s,1H). |

Beispiele zur Herstellung der Endprodukte:

Beispiel 1

D-α-[3-(4-Hydroxy-2-{p-(3',4',5',6'-tetrahydro-pyrimidin-2'-yl)-aminosulfonyl-anilino}-5-pyrimidinyl)-ureido]-p-hydroxybenzylpenicillin-Natrium

1,0 g (2,75 mmol) 5-Amino-4-hydroxy-2-{p-(3',4',5',6'-tetrahydro-pyrimidin-2'-yl)-aminosulfonyl-anilino}-pyrimidin werden in 50 ml wasserfreiem Tetrahydrofuran suspendiert und nach Zugabe von 5 ml Trimethylsilyldiäthylamin 2,5 Stunden bei Raumtemperatur gerührt. Anschließend wird bei 30°C zur Trockne eingedampft und 30 Minuten im Hochvakuum getrocknet. Der Rückstand wird in 50 ml wasserfreiem Tetrahydrofuran aufgenommen und bei 0-5°C zu 3,4 ml einer Phosgenlösung getropft, die 20 g Phosgen in 250 ml wasserfreiem Tetrahydrofuran enthält. Es wird 30 Minuten bei Raumtemperatur gerührt und dann bei maximal 30°C zur Trockne eingedampft. Der Rückstand wird in 40 ml Tetrahydrofuran aufgenommen (Lösung I).

1,1 g (2,6 mmol) Amoxycillin-Trihydrat werden in 100 ml 80%igem wässrigem Tetrahydrofuran suspendiert und durch Einstellen des pH-Wertes auf 8,3 (Triäthylamin) in Lösung gebracht. Bei 5 bis 10°C wird Lösung I zugetropft und der pH-Wert durch Triäthylamin-Zugabe bei 7,5 gehalten. Es wird eine Stunde bei Raumtemperatur gerührt, 100 ml Wasser zugegeben und das Tetrahydrofuran bei 30°C im Vakuum entfernt. Die verbleibende wässrige Lösung wird unter Kühlung mit 2N-Salzsäure auf pH 2,3 eingestellt, der Niederschlag abzentrifugiert, mit Wasser gewaschen und getrocknet (1,6 g). Das Produkt wird in Methanol suspendiert und 0,55 g (3,3 mmol) Natrium-2-äthylhexanoat zugegeben. Nach Zugabe von Diäthyläther erhält man 1,3 g (62,5 % der Theorie) der Titelverbindung als farbloses Pulver.

NMR-Spektrum (DMSO + $CD_3OD$) Signale bei ppm (80 MHz): 1,4-1,8(d+m,8H), 3,2(s,breit,4H), 4,05(s,1H), 5,4(m,3H), 6,75/7,25(q,4H), 7,75(q,4H), 8,3(s,1H).

IR-Spektrum: 1765 $cm^{-1}$.

Auf analoge Weise wurden die in der folgenden Tabelle zusammengefaßten Verbindungen der allgemeinen Formel I hergestellt:

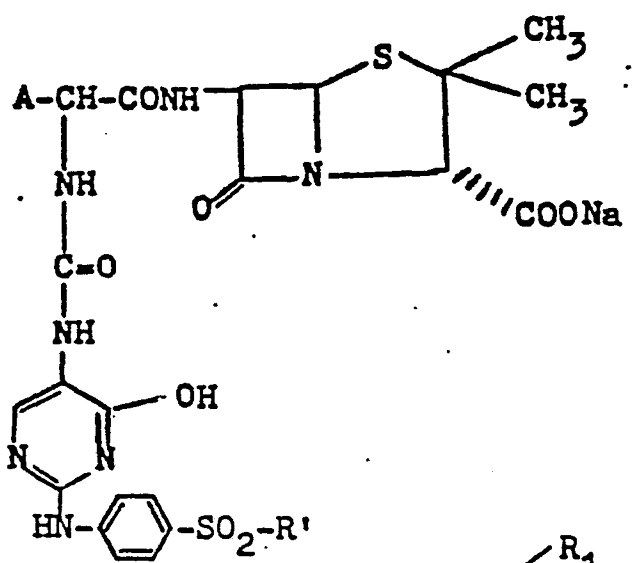

in der R' die Gruppe 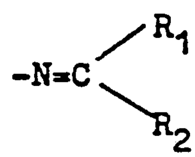 oder

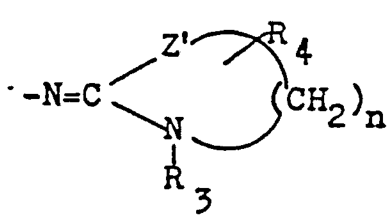 mit den eingangs erwähnten Bedeutungen

für $R_1$, $R_2$, $R_3$, $R_4$, Z' und n darstellt,

| Beisp. | A | R' | IR-Spekt. $cm^{-1}$ | NMR-Spektrum (DMSO + $CD_3OD$) Signale bei ppm (80 MHz): |
|---|---|---|---|---|
| 2) | 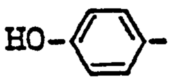 | 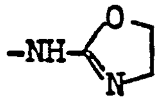 | 1760 | 1,5(d,6H), 3,6(m,2H), 4,05 (s,1H), 4,4(m,2H), 5,4(m,3H), 6,7/7,2(q,4H), 7,75(q,4H), 8,3(s,1H). |
| 3) | 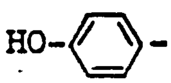 | 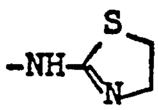 | 1765 | 1,55(d,6H), 3,3-3,7(m,4H), 4,05(s,1H), 5,45(m,3H), 6,75/7,25(q,4H), 7,8(q,4H), 8,3(s,1H). |
| 4) | 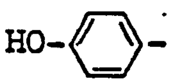 | 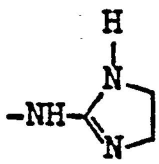 | 1765 | 1,5(d,6H), 3,45(s,4H), 4,05 (s,1H), 5,4(m,3H), 6,75/ 7,2(q,4H), 7,7(s,4H), 8,3 (s,1H). |
| 5) | 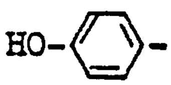 | 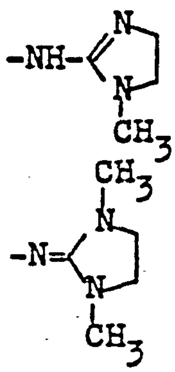 | 1760 | 1,55(d,6H), 2,8(s,3H), 3,5 (s,4H), 4,05(s,1H), 5,5(m,3H), 6,8/7,3(q,4H), 7,8(q,4H), 8,35(s,1H). |
| 6) | 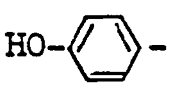 | | 1765 | 1,5(d,6H), 2,9(s,6H), 3,5 (s,4H), 4,0(s,1H), 5,45(m, 3H), 6,75/7,25(q,4H), 7,75 (m,4H), 8,25(s,1H). |
| 7) | 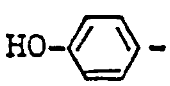 | 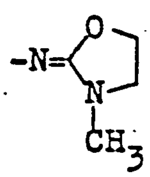 | 1765 | 1,55(d,6H), 2,85(s,3H), 3,6 (m,2H), 4,1(s,1H), 4,5(m,2H), 5,4(m,3H), 6,75/ 7,25(q,4H), 7,75(q,4H), 8,3(s,1H). |
| 8) | 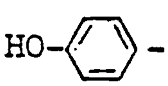 | 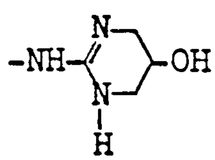 | 1760 | 1,55(d,6H), 3,3(m,4H), 4,05 (s,1H), 5,4(m,3H), 6,7/ 7,25 (q,4H), 7,75(q,4H), 8,25(s, 1H). |

| Beisp. | A | R' | IR-Spekt. $cm^{-1}$ | NMR-Spektrum (DMSO + $CD_3OD$) Signale bei ppm (80 MHz): |
|---|---|---|---|---|
| 9) | 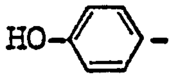 | 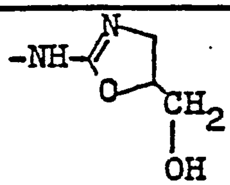 | 1765 | 1,5(d,6H), 3,6(m,4H), 4,05 (s,1H), 4,8(m,1H), 5,5(m,3H), 6,8/7,25(q,4H), 7,8(q,4H), 8,3(s,1H). |
| 10) | 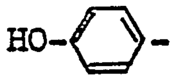 | 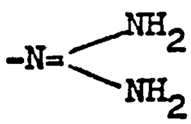 | 1770 | 1,5(d,6H), 4,05(s,1H), 5,4 (m,3H), 6,75/7,2(m,4H), 7,75(m,4H), 8,3(s,1H). |
| 11) | 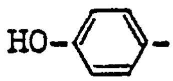 | 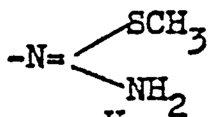 | 1760 | 1,55(d,6H), 2,25(s,3H), 4,0 (s,1H), 5,4(m,3H), 6,7/7,25 (q,4H), 7,75(q,4H), 8,3(s,1H). |
| 12) | 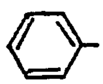 | 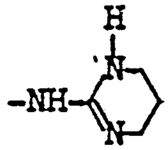 | 1765 | 1,45-1,8(d +m,8H), 3,2(m,4H), 4,05(s,1H), 5,45(m,3H), 7,40-7,80(m,9H), 8,3(s,1H). |
| 13) | 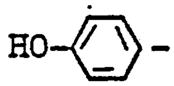 | 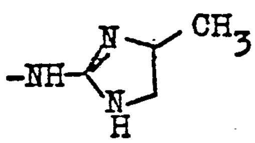 | 1770 | 1,15(d,3H), 1,55(d,6H), 3,0-3,9(m,3H), 4,05(s,1H), 5,4 (m,3H), 6,7(d,2H), 7,2(d,2H), 7,7(q,4H), 8.25(s,1H) |
| 14) | 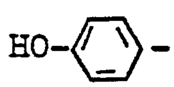 | 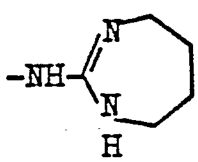 | 1770 | 1,1-1,7 (m,10H), 3,15(m,4H), 4,05(s,1H), 5,4(m,3H), 6,7 (d,2H), 7,2(d,2H), 7,7(q,4H), 8,2(s,1H). |
| 15) | 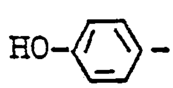 | 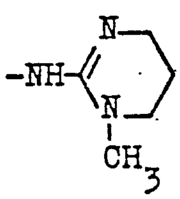 | 1775 | 1,4-1,9(d+m,8H), 2,9(s,3H), 3,2(m,4H), 4,0(s,1H), 5,4 (m,3H), 6,65(d,2H), 7,2(d,2H), 7,65(q,4H), 8,2(s,1H) |
| 16) | 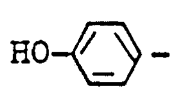 | 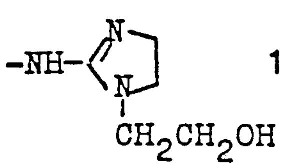 | 1770 | 1,55(d,6H), 3,2-3,7(m,8H), 5,4(m,3H), 6,7(d,2H), 7,2 (d,2H), 7,7 (s breit, 4H), 8,25(s,1H). |

Beispiel 17

D-α-[3-(4-Hydroxy-2-p-(2'-hydroxyäthyl)-aminosulfonyl-anilino-5-pyrimidinyl)-ureido]-p-hydroxybenzyl-penicillin-Natrium

980 mg (3 mMol) 5-Amino-4-hydroxy-2-p-(2'-hydroxyäthyl)-aminosulfonyl-anilino-pyrimidin werden in 50 ml wasserfreiem Tetrahydrofuran suspendiert und nach Zugabe von 5 ml Trimethylsilyldiäthylamin 2,5 Stunden bei Raumtemperatur gerührt. Anschließend wird bei 30°C zur Trockne eingedampft und 30 Minuten im Hochvakuum getrocknet. Der Rückstand wird in 50 ml wasserfreiem Tetrahydrofuran aufgenommen und bei 0 bis 5°C zu 3,75 ml einer Phosgenlösung getropft, die 20 g Phosgen in 250 ml wasserfreiem Tetrahydrofuran enthält. Es wird 30 Minuten bei Raumtemperatur gerührt und dann bei maximal 30°C zur Trockne eingedampft. Der Rückstand wird in 40 ml Tetrahydrofuran aufgenommen (Lösung I).

1,3 g (3,1 mMol) Amoxycillin-Trihydrat werden in 100 ml 80%igem wäßrigen Tetrahydrofuran suspendiert und durch Einstellen des pH-Wertes auf 8,3 (Triäthylamin) in Lösung gebracht. Bei 5 bis 10°C wird Lösung I zugetropft und der pH-Wert durch Triäthylamin-Zugabe bei 7,5 gehalten. Es wird eine Stunde bei Raumtemperatur gerührt, 100 ml Wasser zugegeben und das Tetrahydrofuran bei 30°C im Vakuum entfernt. Die verbleibende wäßrige Lösung wird unter Kühlung mit 2N-Salzsäure auf pH 2,7 eingestellt, der Niederschlag wird abgesaugt, mit Wasser gewaschen und getrocknet (1,6 g).

Das Produkt wird in Methanol suspendiert und 0,55 g (3,3 mmol) Natrium-2-äthylhexanoat zugegeben. Nach Zugabe von Diäthyläther erhält man 1,3 g (59 % der Theorie) der Titelverbindung als farbloses Pulver.
NMR-Spektrum (DMSO + $CD_3OD$) Signale bei ppm:
1,55(d,6H), 3,2(m,2H), 3,55(m,2H), 4,0(s,1H), 5,40(q,2H), 5,45(s,1H), 6,70(d,2H), 7,25(d,2H), 7,80(q,4H), 8,35(s,1H).
IR-Spektrum: 1765, 1660, 1610, 1155 $cm^{-1}$.

Auf analoge Weise wurden die in der folgenden Tabelle zusammengefaßten Verbindungen der Formel

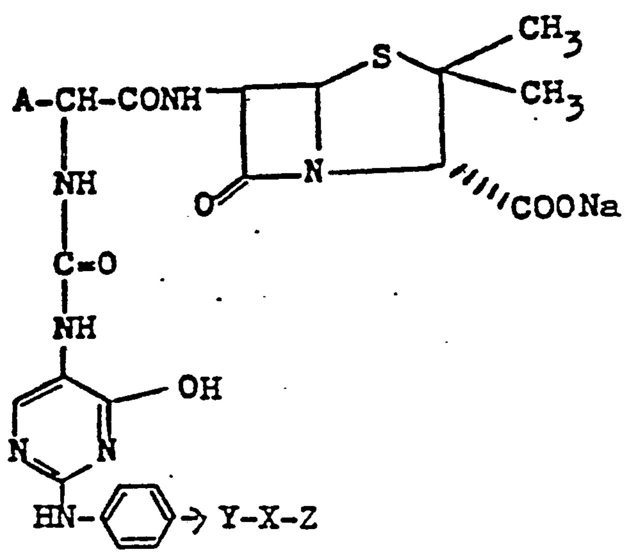

hergestellt.

| Beisp. | A | →Y-Z-X | IR-Spekt. $cm^{-1}$ | NMR-Spektrum (DMSO+ $CD_3OD$) Sig. bei ppm |
|---|---|---|---|---|
| 18) | 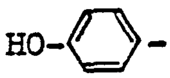 | $-SO_2N(CH_2CH_2OH)_2$ | 1765 1655 1150 | 1,55(d,6H), 3,20(m,4H), 3,65(m,4H), 3,95(s,3H), 5,45(q,2H+s,1H), 6,75 (d,2H), 7,25(d,2H), 7,8(s,4H), 8,30(s,1H). |
| 19) | 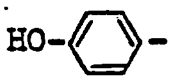 | 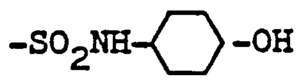 | 1760 1155 | 1,55(d,6H), 1,2-1,9(m, 8H), 3,1(m,breit,1H), 3,6(m,1H), 3,95(s,1H), 5,40(q,2H), 5,50(s,1H), 6,75(d,2H), 7,3(d,2H), 7,75(q,4H), 8,33(s,1H). |
| 20) | 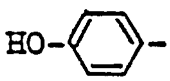 | 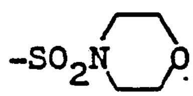 | 1765 1660 1605 1105 | 1,55(d,6H), 2,8(m,4H), 3,6(m,4H), 3,95(s,1H), 5,40(m,3H), 6,8(d,2H), 7,3(d,2H), 7,8(s,4H), 8,32(s,1H). |
| 21) | 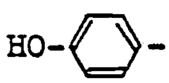 | $-SO_2NHCH_2CONH_2$ | 1765 1150 | 1,55(d,6H), 3,45(s, breit,2H), 3,95(s,1H), 5,45(m,3H), 6,8(d,2H), 7,35(d,2H), 7,8(s,4H), 8,3(s,1H). |
| 22) | 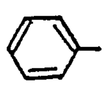 | $-SO_2NHCH_2CONH_2$ | 1760 1155 | 1,55(d,6H), 3,40(s,2H), 4,0(s,1H), 5,45(m,3H), 7,35(s,breit,5H), 7,75 (s,4H), 8,32(s,1H). |

| Beisp. | A | Y-Z-X | IR-Spekt. $cm^{-1}$ | NMR-Spektrum (DMSO + $CD_3OD$) Sig. bei ppm |
|---|---|---|---|---|
| 23) | 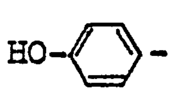 | 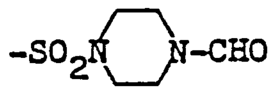 | 1765<br>1660<br>1150 | 1,55(d,6H), 2,95(m,4H), 3,50(m,4H), 4,0(s,1H), 5,40(q,2H), 5,45(s,1H), 6,8(d,2H), 7,3(d,2H), 7,8(q,4H), 8,0(s,1H), 8,35(s,1H). |
| 24) | 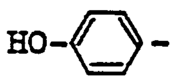 | $-SO_2NHCH_2CH_2NH$<br>\|<br>$COCH_3$ | 1765<br>1660<br>1610<br>1150 | 1,55(d,6H), 1,8(s,3H), 2,8(m,2H), 3,3(m,2H), 4,05(s,1H), 5,45(q,2H), 5,50(s,1H), 6,75(d,2H), 7,30(d,2H), 7,75(q,4H), 8,33(s,1H). |
| 25) | 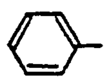 | $-SO_2NHCH_2CH_2NHCOCH_3$ | 1765<br>1155 | 1,50(d,6H), 1,85(s,3H), 2,75(m,2H), 3,25(m,2H), 4,0(s,1H), 5,45(m,3H), 7,40(s,breit,5H), 7,75 (q,4H), 8,31(s,1H). |
| 26) | 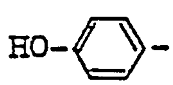 | $-SO_2NHCH_2CH_2COOH$ | 1765<br>1610<br>1155 | 1,55(d,6H), 2,35(t,2H), 2,90(t,2H), 3,95(s,1H), 5,45(m,3H), 6,8(d,2H), 7,3(d,2H), 7,8(s,4H), 8,3(s,1H). |

## Beispiel 27

**D,L-α-[3-(2-p-(2'-Hydroxyäthyl)-aminosulfonyl-anilino-5-pyrimidinyl)-ureido]-2-thienyl-acetamido-penicillansäure-Natriumsalz**

1.10 g der Ureidocarbonsäure des Beispiels D (2 mMol), gelöst in 50 ml Dimethylformamid, werden mit 200 mg N-Methylmorpholin versetzt. Bei -15 bis -20°C werden 205 mg (2 mmol) Chlorameisensäureäthylester zugefügt und 10 Minuten bei dieser Temperatur gerührt (Lösung I).

460 mg (2,1 mmol) 6-Aminopenicillansäure, suspendiert in 50 ml Methylenchlorid, werden mit Triäthylamin bis zur Lösung gerührt. Diese Lösung wird bei -20°C zu Lösung A gegeben und anschließend 30 Minuten bei -20°C und zwei Stunden bei Raumtemperatur gerührt.

Man gibt 100 ml Wasser zu, stellt den pH-Wert auf 7,2 und extrahiert mit Essigester. Die wäßrige Phase wird unter Eiskühlung auf pH 2,8 eingestellt (2 N-Salzsäure), der Niederschlag abgesaugt, mit Eiswasser gewaschen und getrocknet. Eine methanolische Suspension dieses Produkts wird mit Natrium-2-äthylhexanoat versetzt und das Natriumsalz mit Diäthyläther gefällt. Man erhält 840 mg (54 %) der Titelverbindung als farbloses Pulver.

NMR-Spektrum (DMSO + $CD_3OD$) Signale bei ppm:
1,55(d,6H), 3,25(m,2H), 3,65(m,2H), 4,0(s,1H), 5,4(q,2H), 5,65(s,1H), 7,0(m,2H), 7,4(d,1H), 7,8(q,4H), 8,32(s,1H).
IR-Spektrum: 1765 $cm^{-1}$;

Auf analoge Weise wurden die in der folgenden Tabelle zusammengefaßten Verbindungen der Formel

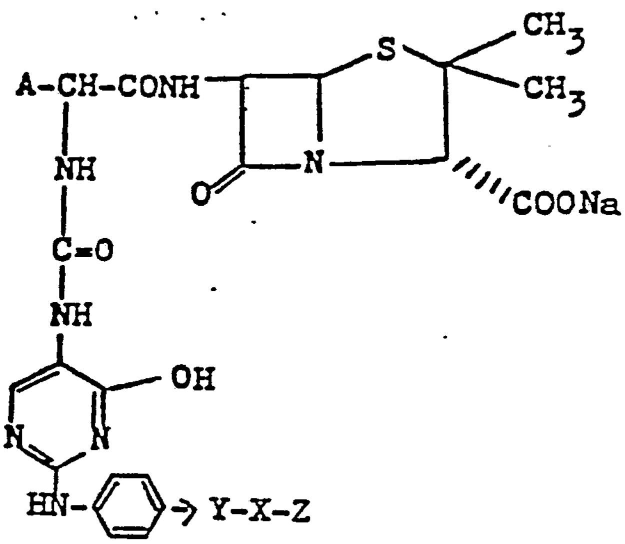

erhalten:

| | A | Y-X-Z | IR-Spekt. $cm^{-1}$ | NMR-Spektrum (DMSO+ $CD_3OD$) Sig. bei ppm |
|---|---|---|---|---|
| 28) | 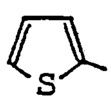 | $-SO_2NH(CH_2)_3CONH_2$ | 1765<br>1150 | 1,55(d,6H), 1,80(m,2H), 2,3(m,2H), 2,85(m,2H), 4,05(s,1H), 5,45(q,2H), 5,70(s,breit,1H), 7,0 (m,2H), 7,4(d,1H), 7,80(q,4H), 8,32(s,1H). |
| 29) | 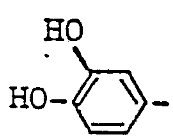 | $-SO_2NHCH_2CH_2OH$ | 1765<br>1155 | 1,55(d,6H), 3,20(m,2H), 3,55(m,2H), 5,45(m,3H), 6,7(s,2H), 6,85(s,1H), 7,75(q,4H), 8,33(s,1H). |

Beispiel 30

D-α-/3-(4-Hydroxy-2-{p-(3',4',5',6'-tetrahydro-pyrimidin-2'-yl)-aminosulfonyl-anilino}-5-pyrimidinyl)-ureido7-p-hydroxybenzylpenicillin-Natrium

1,8 g (3,2 mmol) D-α-/3-(4-Hydroxy-2-{p-(3',4',5',6'-tetrahydropyrimidin-2'-yl)-aminosulfonyl-anilino}-5-pyrimidinyl)-ureido7-p-hydroxy-phenylglycin in 80 ml Dimethylformamid werden mit 323 mg N-Methylmorpholin versetzt. Bei -15 bis -20°C werden 345 mg (3,2 mmol) Chlorameisensäureäthylester zugefügt und 30 Minuten bei dieser Temperatur gerührt (Lösung I).

690 mg (3,2 mmol) 6-Aminopenicillansäure, suspendiert in 50 ml Methylenchlorid, werden mit 2 g (20 mmol) Triäthylamin bis zur Lösung gerührt. Diese Lösung wird bei -20°C zu Lösung A gegeben und anschließend 30 Minuten bei -20°C und zwei Stunden bei Raumtemperatur gerührt.

Man gibt 200 ml Wasser zu, stellt den pH-Wert auf 7,2 und extrahiert mit Essigester. Die wässrige Phase wird unter Eiskühlung auf pH 2,3 eingestellt (2 N-Salzsäure), der Niederschlag abzentrifugiert, mit Eiswasser gewaschen und getrocknet. Eine methanolische Suspension dieses Produkts wird mit Natrium-2-äthylhexanoat versetzt und das Natriumsalz mit Diäthyläther gefällt. Man erhält 1,7 g (68 %) der Titelverbindung als farbloses Pulver.
NMR-Spektrum (DMSO + $CD_3OD$) Signale bei ppm (80 MHz):
1,4-1,8(d+m,8H), 3,25(m,4H), 4,05(s,1H), 5,4(m,3H), 6,70/7,2 (q,4H), 7,8(q,4H), 8,3(s,1H).
IR-Spektrum: 1765 $cm^{-1}$.

Auf analoge Weise wurden die in der folgenden Tabelle zusammengefaßten Verbindungen der allgemeinen Formel I erhalten:

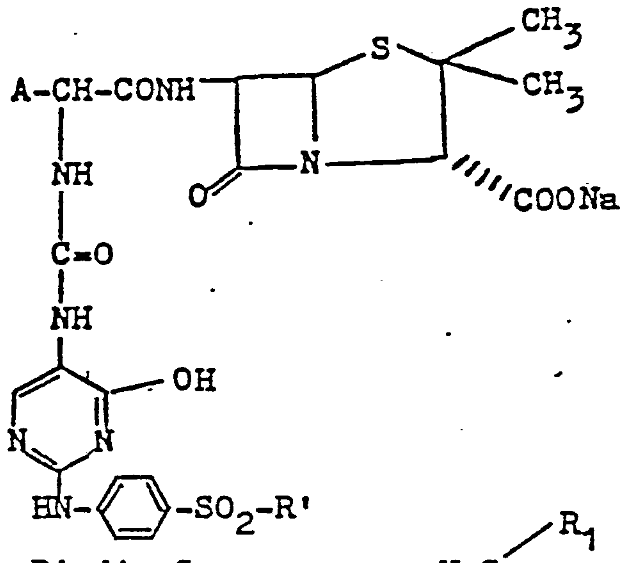

in der R' die Gruppe 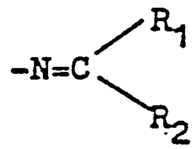 oder

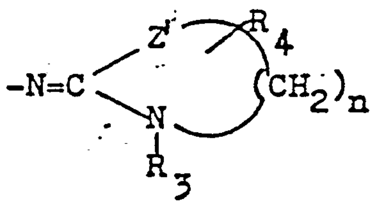 bedeutet,

| Beisp. | A | R' | IR-Spekt. $cm^{-1}$ | NMR-Spektrum (DMSO + $CD_3OD$) Signale bei ppm (80 MHz): |
|---|---|---|---|---|
| 31) | 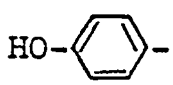 | 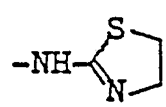 | 1765 | 1,50(d,6H), 3,25-3,7(m,4H), 4,05(s,1H), 5,4(m,3H), 6,75/7,2(q,4H), 7,75(q,4H), 8,3 (s,1H). |
| 32) | 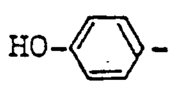 | 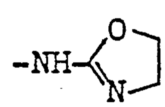 | 1760 | 1,50(d,6H), 3,55(m,2H), 4,05 (s,1H), 4,4(m,2H), 5,45(m,3H), 6,75 / 7,25(q,4H), 7,8(q,4H), 8,3(s,1H). |
| 33) | 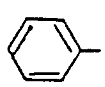 | 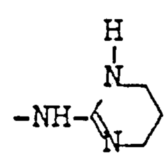 | 1765 | 1,4-1,8(d+m,8H), 3,2(m,4H), 4,05(s,1H), 5,4(m,3H), 7,40-7,80(m,9H), 8,3(s,1H). |

Beispiel 34

D-α-[3-(4-Hydroxy-2-p-(2'-hydroxyäthyl)-sulfinyl-anilino-5-pyrimidinyl)-ureido]-p-hydroxybenzyl-penicillin-Natrium

260 mg (0,88 mMol) 5-Amino-4-hydroxy-2-p-(2'-hydroxyäthyl)-sulfinyl-anilino-pyrimidin werden in 10 ml wasserfreiem Tetrahydrofuran suspendiert, 2ml N,N-Diäthyltrimethylsilylamin werden zugesetzt und die Mischung 30 Minuten unter Rückfluß gekocht. Nach dem Absaugen von einer geringen Menge unlöslicher Nebenprodukte wird die Lösung im Vakuum zur Trockne eingedampft. Der Rückstand wird in 15 ml absolutem Tetrahydrofuran gelöst und unter Eiskühlung zu einer Lösung von 87 mg Phosgen (0,88 mMol) in 8,5 ml Tetrahydrofuran getropft. Die Lösung wird im Vakuum etwa auf das halbe Volumen eingedampft und unter Rühren und Eiskühlung zu einer Lösung von 406 mg Amoxicillin-Trihydrat in 8 ml Tetrahydrofuran, 2 ml Wasser und so viel Triäthylamin, daß der pH-Wert 8,5-8,8 beträgt, zugetropft.

Das Reaktionsgemisch wird noch 1 Stunde bei Raumtemperatur gerührt. Der pH-Wert wird bei 7,2-7,5 gehalten, falls erforderlich durch Zugabe von Triäthylamin. Nach Zusatz von 20 ml Wasser wird das Tetrahydrofuran im Vakuum abgezogen und unter Eiskühlung und Rühren mit 2n-Salzsäure ein pH-Wert von 2,5 eingestellt. Das angefallene Penicillin wird abgesaugt, mit Wasser gewaschen und im Exsikkator im Hochvakuum getrocknet.

Ausbeute: 230 mg (38 %);

Die Säure wird in einer Lösung von 55 mg Natrium-2-äthylhexanoat in 50 ml Methanol gerührt. Nach Abfiltrieren von wenig unlöslichem Rückstand wird das Natriumsalz mit absolutem Äther gefüllt. Man erhält 160 mg (26 %) der Titelverbindung als farbloses Pulver.

IR-Spektrum: 1760, 1660, 1020 $cm^{-1}$;
NMR-Spektrum (DMSO + $CD_3OD$) Signale bei ppm:
1,55(d,6H), 2,95(m,2H), 3,75(m,2H), 4,05(s,1H), 5,45(m,3H), 6,75(d,2H), 7,30(d,2H), 7,80(q,4H), 8,40(s,1H).

Auf analoge Weise wurden die in der folgenden Tabelle zusammengefaßten Verbindungen der Formel

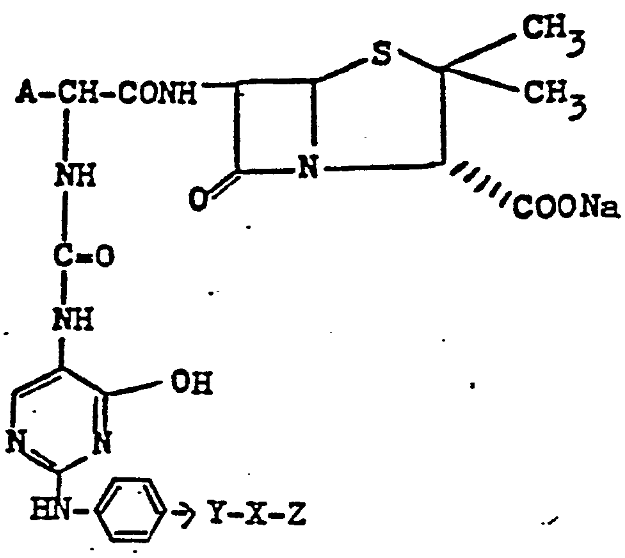

hergestellt:

| Beisp. | A →Y-Z-X | IR-Spekt. $cm^{-1}$ | NMR-Spektrum (DMSO + $CD_3OD$) Sig. bei ppm |
|---|---|---|---|
| 35) | 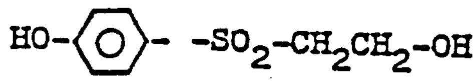 | 1765<br>1660<br>1600 | 1,5(d,6H), 3,5(m,4H), 4,0(s,1H), 5,4(m,3H), 6,75(m,2H), 7,2(m,2H), 7,7(d,2H), 8,0(m,2H), 8,35(s,1H). |
| 36) | 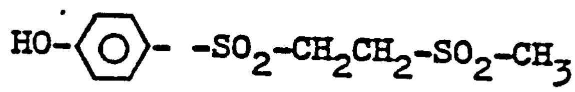 | 1765<br>1660<br>1600<br>1305<br>1140 | 1,55(d,6H), 3,1(s,3H), 3,5(m,4H), 4,05(s,1H), 5,4(m,3H), 6,75(d,2H), 7,25(d,2H), 7,9(q,4H), 8,4(s,1H). |
| 37) | 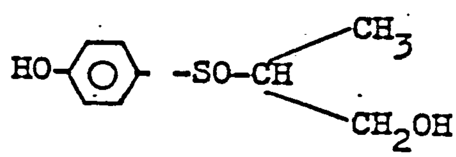 | 1765<br>1655<br>1600 | 0,95(t,3H), 1,55(d,6H), 2,9(m,1H), 3,65(m,2H), 4,1(s,1H), 5,4(m,3H), 6,75(d,2H), 7,3(d,2H), 7,5(d,2H), 7,9(d,2H), 8,3(s,1H). |
| 38) | 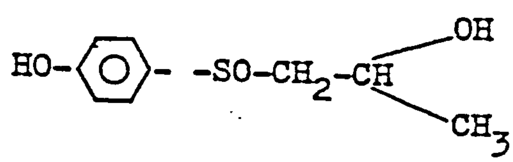 | 1770<br>1660<br>1600 | 1,2(m,3H), 1,55(d,6H), 2,9(m,2H), 3,8(m,1H), 4,05(s,1H), 5,4(m,3H), 6,75(d,2H), 7,3(d,2H), 7,6(d,2H), 7,95(d,2H), 8,3(s,1H). |
| 39) | 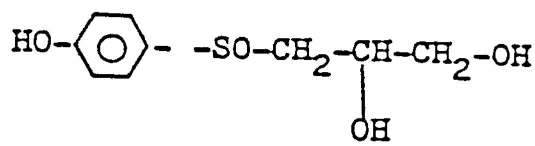 | 1765<br>1660<br>1600 | 1,55(d,6H), 2,95(m,2H), 3,45(m,3H), 3,65(m,1H), 4,05(s,1H), 5,4(m,3H), 6,75(d,2H), 7,5(breites m,4H), 8,0(d,2H), 8,3 (s,1H). |

Die Verbindungen der allgemeinen Formel I oder I', lassen sich in die üblichen pharmazeutischen Anwendungsformen, wie Tabletten, Dragées, Kapseln oder Ampullen einarbeiten. Die Einzeldosis beträgt bei Erwachsenen im allgemeinen zwischen 50 und 1000 mg, vorzugsweise 100 bis 500 mg, die Tagesdosis zwischen 100 und 4000 mg, vorzugsweise 250 bis 2000 mg.

Beispiel I

Tabletten enthaltend D-α-[3-(2-p-(2'-Hydroxyäthyl)-sulfinyl-anilino-4-hydroxy-5-pyrimidinyl)-ureido]-p-hydroxybenzyl-penicillin-Natrium

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepreßt, derart, daß jede Tablette 200 mg Wirkstoff enthält.

Beispiel II

Dragées enthaltend D-α-[3-(2-p-(2'-Hydroxyäthyl)-sulfinyl-anilino-4-hydroxy-5-pyrimidinyl)-ureido]-p-hydroxybenzyl-penicillin-Natrium

Analog Beispiel I werden Tabletten gepreßt, die anschließend in üblicher Weise mit einem Überzug bestehend aus Zucker, Kartoffelstärke, Talk und Tragant überzogen werden.

Beispiel III

Kapseln enthaltend D-α-[3-(2-p-(2'-Hydroxyäthyl)-sulfinylanilino-4-hydroxy-5-pyrimidinyl)-ureido]-p-hydroxybenzylpenicillin-Natrium

5 kg Wirksubstanz werden in üblicher Weise in Hartgelatinekapseln gefüllt, derart, daß jede Kapsel 500 mg des Wirkstoffes enthält.

Beispiel IV

Tabletten enthaltend D-α-[3-(4-Hydroxy-2-{p-(3',4',5',6'-tetrahydro-pyrimidin-2'-yl)-aminosulfonylanilino}-5-pyrimidinyl)-ureido]-p-hydroxy-benzylpenicillin-Natrium

In einem aspetischen Bereich wurde 251 g Wirkstoff in 200 ml destilliertem Wasser zur Injektion aufgelöst. Die Lösung wurde durch ein Millipore-Filter (Porengröße 0,22 µm, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 2 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragées, Kapseln und Ampullen erhältlich, die einen oder mehrere der übrigen Wirkstoffe der Formel I oder die physiologisch unbedenklichen Salze dieser Verbindungen enthalten.

## P a t e n t a n s p r ü c h e

1. Neue Penicilline der allgemeinen Formel

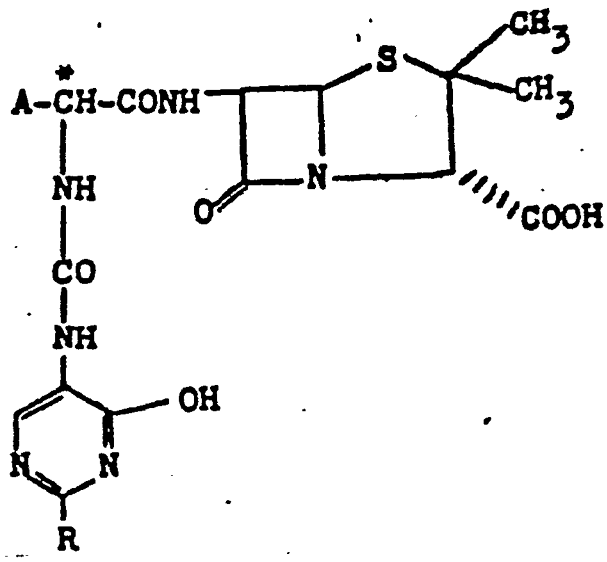

,(I)

oder

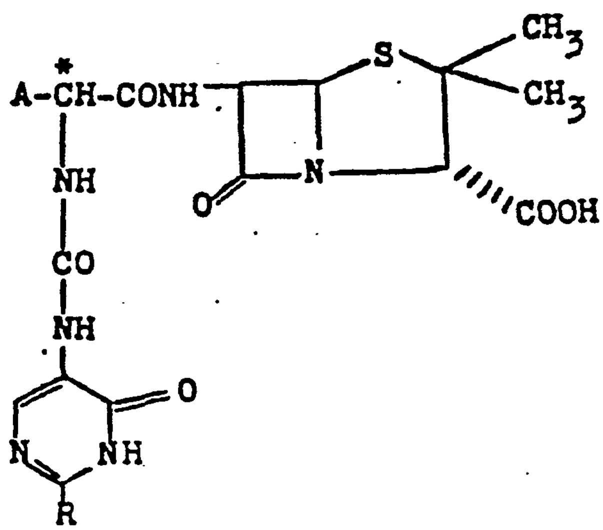

,(I')

in welchen A und R die folgenden Bedeutungen besitzen:

A die Phenyl-, 4-Hydroxyphenyl-, 2-Thienyl- oder 3,4-Dihydroxyphenylgruppe und

R 1. eine Gruppe der allgemeinen Formel IIa

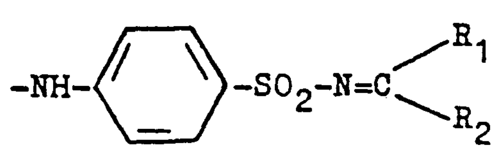

,(IIa)

in der die Reste $R_1$ und $R_2$ gleich oder verschieden sind und die Methylmercaptogruppe oder die Aminogruppe bedeuten, oder

2. eine Gruppe der allgemeinen Formel IIb

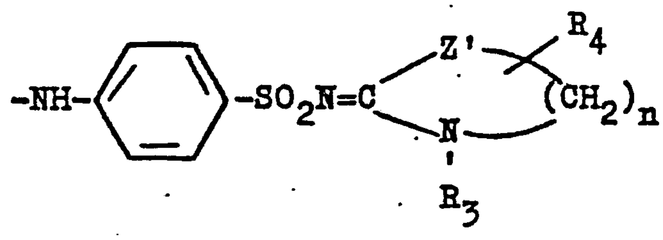

,(IIb)

in der
Z' für -O-, -S- oder $=NR_3$ und
$R_3$ für ein Wasserstoffatom oder eine Alkylgruppe mit 1 bis 3 Kohlenstoffatomen steht, die gegebenenfalls durch eine Hydroxygruppe substituiert sein kann und
$R_4$ ein Wasserstoffatom, die Hydroxy-, Hydroxymethyl- oder Methylgruppe bedeutet

und n die Zahlen 2, 3 oder 4 darstellt, wobei die Gruppen der Formeln IIa und IIb, wenn einer oder beide Reste $R_1$ und $R_2$ eine Aminogruppe bedeuten oder wenn $R_3$ Wasserstoff darstellt, auch in den folgenden tautomeren Formen der Formeln IIa' bzw. IIb' vorliegen können:

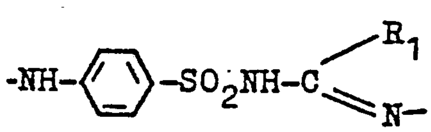

oder

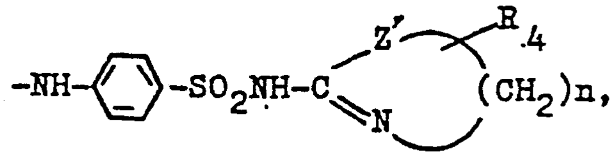

IIa' IIb'

oder

3. eine Gruppe der allgemeinen Formel

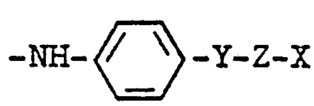

in der
Y eine Gruppe der Formel $-SO_2-NH-$, $-SO-$ oder $-SO_2-$ und
Z eine geradkettige oder verzweigte Alkylengruppe mit 1 bis 3 Kohlenstoffatomen und
X eine Hydroxy-, Aminocarbonyl-, Aminosulfonyl-, Formylamino-, Acetylamino-, Amino-, Methylsulfinyl-, Methylsulfonyl- oder eine Aminocarbonsäuregruppe

der Formel

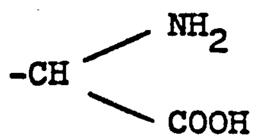

sein können,

oder es bedeutet -Y-Z-X
die Gruppe $-SO_2N(CH_2CH_2OH)_2$, die Gruppe 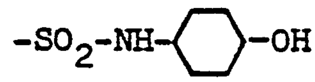

die Gruppe 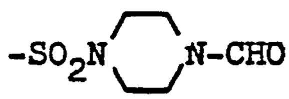 oder 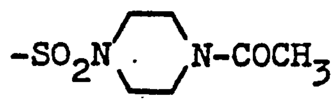

die Gruppe 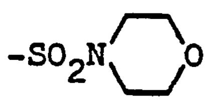, oder 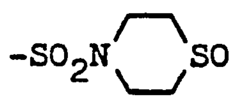 oder

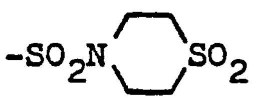, oder die Gruppe

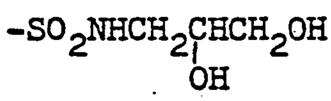 oder 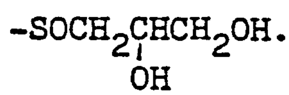.

und deren physiologisch verträgliche Salze mit anorganischen oder organischen Basen.

2. Neue Penicilline der allgemeinen Formel I bzw. I', gemäß Anspruch 1, dadurch gekennzeichnet, daß

A die Phenyl- oder p-Hydroxyphenylgruppe darstellt und
R die folgenden Bedeutungen besitzt:

1. eine Gruppe der Formel

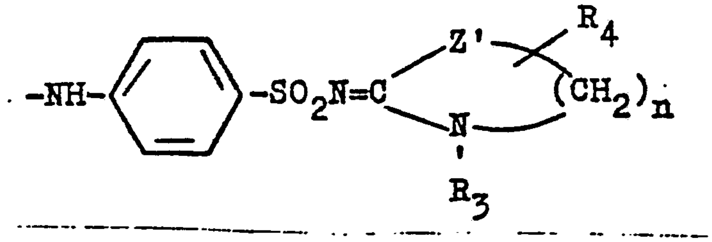 ,(IIb)

in der
n die Zahlen 2, 3 oder 4 bedeutet,
Z die Gruppe $=NR_3$ darstellt, wobei
$R_3$ und $R_4$ ein Wasserstoffatom oder die Methylgruppe bedeuten, oder

2. eine Gruppe der Formel

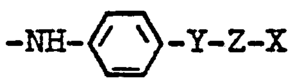

in der Y und Z die im Anspruch 1 genannten Bedeutungen innehaben und X die Hydroxy-, Aminocarbonyl-, Aminosulfonyl-, Acetylamino-, Methylsulfinyl- oder die Methylsulfonylgruppe darstellt oder in der -Y-Z-X eine der Gruppen

$-SO_2N(CH_2CH_2OH)_2$, die Gruppe 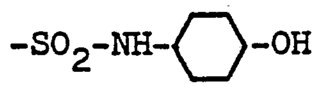

die Gruppe 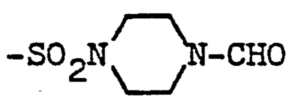

oder die Gruppe $-SO_2NHCH_2CHCH_2OH$ oder
OH

$-SOCH_2CHCH_2OH$ bedeutet,
OH

und ihre physiologisch verträgliche Salze mit anorganischen oder organischen Basen.

3. Neue Penicilline der allgemeinen Formel I bzw. I', gemäß den Ansprüchen 1 und 2, dadurch gekennzeichnet, daß diese die D=R-Konfiguration haben.

4. Als neue Verbindungen
D-α-/3-(4-Hydroxy-2-p-(4',5'-dihydro-oxazol-2'-yl)-amino-sulfonylanilino-5-pyrimidinyl)-ureido7-p-hydroxybenzyl-penicillansäure,

D-α-/3-(4-Hydroxy-2-p-(2'-hydroxyisopropyl)-sulfinylanilino-5-pyrimidinyl)-ureido7-p-hydroxybenzylpenicillansäure und

D-α-/3-(4-Hydroxy-2-p(aminocarbonylmethylamino)-sulfonyl-anilino-5-pyrimidinyl)-ureido7-p-hydroxybenzylpenicillan-säure

und deren physiologisch verträgliche Salze mit anorganischen oder organischen Basen.

5. Arzneimittel, enthaltend eine oder mehrere Verbindungen der allgemeinen Formeln I bzw. I' oder ihre physiologisch verträglichen Salze mit anorganischen oder organischen Basen neben den üblichen Träger- und/oder Hilfsstoffen.

6. Verfahren zur Herstellung neuer Penicilline der allgemeinen Formeln

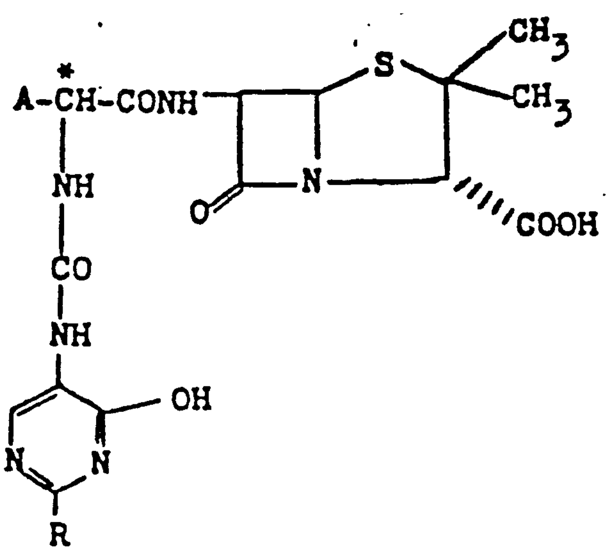

,(I)

oder

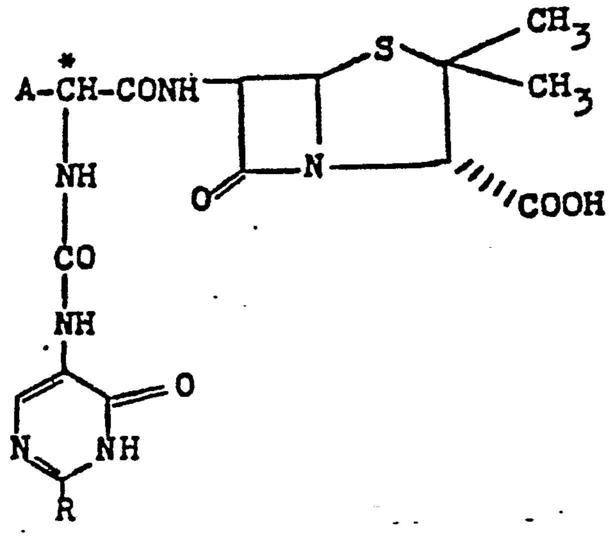

,(I')

in welchen A und R die folgenden Bedeutungen besitzen:

A die Phenyl-, 4-Hydroxyphenyl-, 2-Thienyl- oder 3,4-Dihydroxyphenylgruppe und

R

1. eine Gruppe der allgemeinen Formel IIa

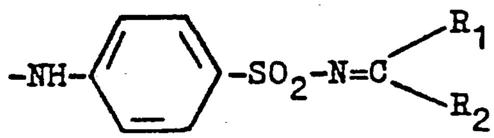

,(IIa)

in der die Reste $R_1$ und $R_2$ gleich oder verschieden sind und die Methylmercaptogruppe oder die Aminogruppe bedeuten, oder

2. eine Gruppe der allgemeinen Formel IIb

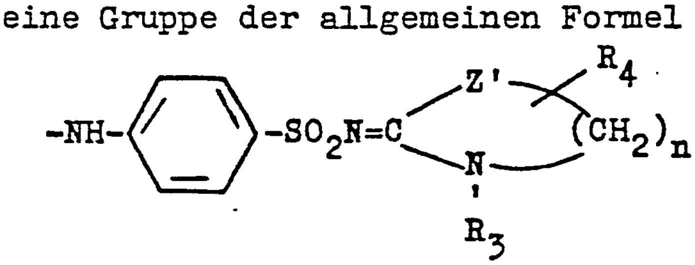

,(IIb)

in der
Z' für -O-, -S- oder $=NR_3$ und
$R_3$ für ein Wasserstoffatom oder eine Alkylgruppe mit 1 bis 3 Kohlenstoffatomen steht, die gegebenenfalls durch eine Hydroxygruppe substituiert sein kann und
$R_4$ ein Wasserstoffatom, die Hydroxy-, Hydroxymethyl- oder Methylgruppe bedeutet

und n die Zahlen 2, 3 oder 4 darstellt, wobei die Gruppen der Formeln IIa und IIb, wenn einer oder beide Reste $R_1$ und $R_2$ eine Aminogruppe bedeuten oder wenn $R_3$ Wasserstoff darstellt, auch in den folgenden tautomeren Formen der Formeln IIa' bzw. IIb' vorliegen können:

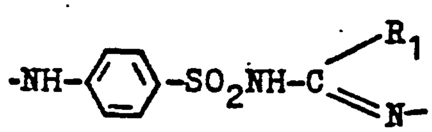

oder

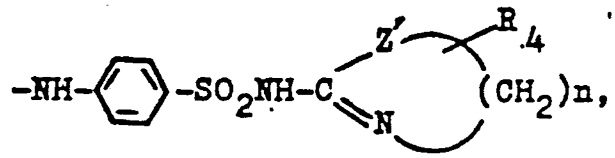

IIa' IIb'

oder

3. eine Gruppe der allgemeinen Formel

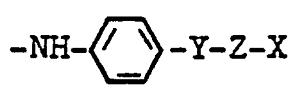

in der
Y eine Gruppe der Formel $-SO_2-NH-$, $-SO-$ oder $-SO_2-$ und
Z eine geradkettige oder verzweigte Alkylengruppe mit 1 bis 3 Kohlenstoffatomen und
X eine Hydroxy-, Aminocarbonyl-, Aminosulfonyl-, Formylamino-, Acetylamino-, Amino-, Methylsulfinyl-, Methylsulfonyl- oder eine Aminocarbonsäuregruppe

der Formel

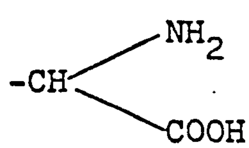

sein können,

oder es bedeutet -Y-Z-X
die Gruppe $-SO_2N(CH_2CH_2OH)_2$, die Gruppe $-SO_2-NH-$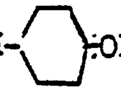-OH

die Gruppe $-SO_2N$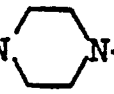N-CHO oder $-SO_2N$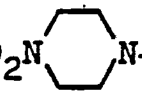$N-COCH_3$ ,

die Gruppe $-SO_2N$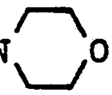O, oder $-SO_2N$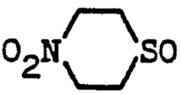SO oder

$-SO_2N$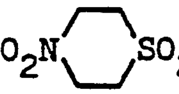$SO_2$, oder die Gruppe

$-SO_2NHCH_2CH(OH)CH_2OH$ oder $-SOCH_2CH(OH)CH_2OH$.

und von deren Salzen mit anorganischen oder organischen Basen, dadurch gekennzeichnet, daß

a) eine Verbindung der allgemeinen Formel III,

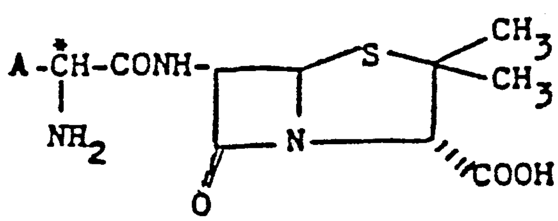
,(III)

in der A wie oben definiert ist, oder ihre Salze oder Ester mit einem Pyrimidinderivat der allgemeinen Formel IV,

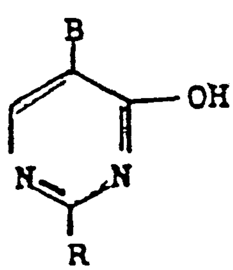
,(IV)

in der R wie oben definiert ist und B die Gruppe -NCO oder ein reaktives Derivat der Gruppe NHCOOH bedeutet, wie z. B.
die Gruppen -NHCOCl, -NHCOBr oder -NH-COO-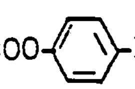$-NO_2$,

wobei die Gruppe NHCOCl besonders bevorzugt ist, oder mit Gemischen von solchen Pyrimidinderivaten der allgemeinen Formel IV, in der B teils die eine und teils die andere der vorstehend genannten Bedeutungen besitzt, in einem Lösungsmittel bei einem pH-Wert zwischen 2,0 und 9,0 bei Temperaturen zwischen -20 und +50°C, gegebenenfalls in Gegenwart einer Base, umgesetzt wird oder

b) Ureidocarbonsäuren der allgemeinen Formel V,

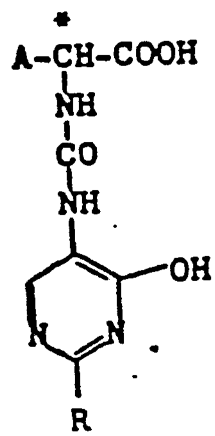

,(V)

in der A und R die oben angegebenen Bedeutungen besitzen, ihre Salze oder reaktiven Derivate, mit der 6-Aminopenicillansäure der Formel VI,

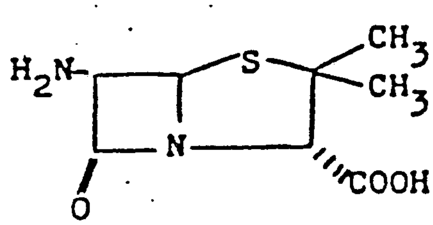

,(VI)

oder ihren anorganischen oder organischen Salzen oder Derivaten, die in die 6-Aminopenicillansäure leicht überführbar sind, in einem Lösungsmittel bei Temperaturen zwischen -40 und +40°C, gegebenenfalls in Gegenwart einer Base, umgesetzt werden und, gewünschtenfalls, so erhaltene Verbindungen der allgemeinen Formel I bzw. I' anschließend mittels anorganischer

oder organischer Basen in ihre Salze übergeführt werden.

7. Verfahren gemäß Anspruch 6a, dadurch gekennzeichnet, daß eine Verbindung der allgemeinen Formel III, oder eines ihrer Salze mit anorganischen oder organischen Basen mit einer Verbindung der allgemeinen Formel IV

a) in Wasser oder in mit Wasser mischbaren Lösungsmitteln in Gegenwart von Wasser in einem pH-Bereich von 6,5 bis 8,0 oder
b) in wasserfreien Lösungsmitteln oder
c) in einem Gemenge aus Wasser und mit Wasser nicht mischbaren Lösungsmitteln in einem pH-Bereich zwischen 6,5 und 8,0

umgesetzt wird, oder daß eine Verbindung der allgemeinen Formel III, in der in der Carboxylgruppe das Proton durch eine Silylgruppe oder eine andere leicht abspaltbare Schutzgruppe ersetzt ist, mit einer Verbindung der allgemeinen Formel IV in einem wasser- und hydroxylgruppenfreien bzw. aprotischen Lösungsmittel, gegebenenfalls in Gegenwart einer Base, umgesetzt wird.

8. Verfahren gemäß Anspruch 6b, dadurch gekennzeichnet, daß als reaktive Derivate der Ureidocarbonsäuren der allgemeinen Formel V deren Säureanhydride, deren reaktive Ester oder reaktive Amide oder deren Säurehalogenide, als Derivate der 6-Aminopenicillansäure der Formel VI ihre Ester, vorzugsweise ihr Trimethylsilylester, Tritylester, p-Nitrobenzylester, Phenacylester oder O,N-Bis-trimethylsilylderivat verwendet werden und die Umsetzung, wenn erforderlich, in Gegenwart einer Base und/oder eines organischen Lösungsmittels und/oder eines Kondensationsmittels erfolgt, und erforderlichenfalls das so erhaltene Produkt der allgemeinen Formel I bzw. I', dessen Carboxylgruppe durch eine Schutzgruppe geschützt ist, anschließend in eine Verbindung der allgemeinen Formel I bzw. I' übergeführt wird, die die freie Carboxylgruppe besitzt.

## Patentansprüche für das Benennungsland Österreich

1. Verfahren zur Herstellung neuer Penicilline der allgemeinen Formeln

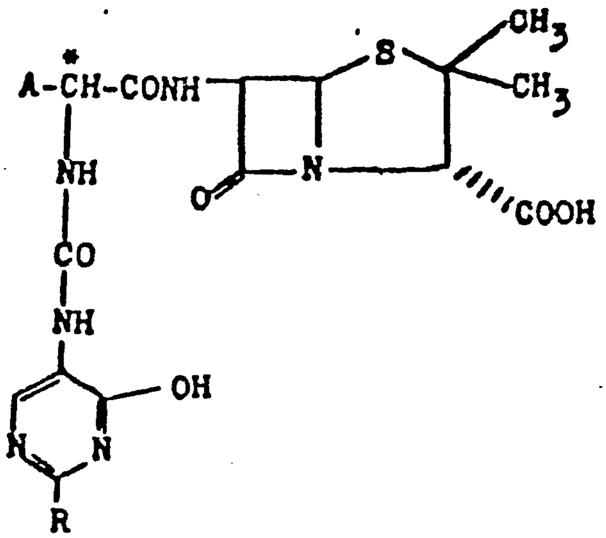

,(I)

oder

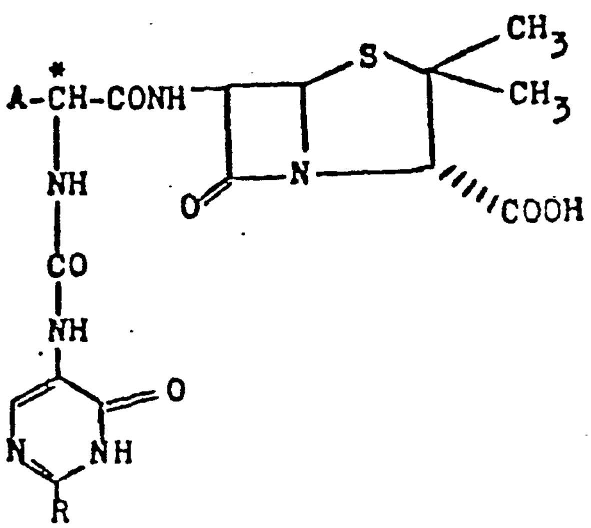

,(I')

in welchen A und R die folgenden Bedeutungen besitzen:

A die Phenyl-, 4-Hydroxyphenyl-, 2-Thienyl- oder 3,4-Dihydroxyphenylgruppe und

R

1. eine Gruppe der allgemeinen Formel IIa

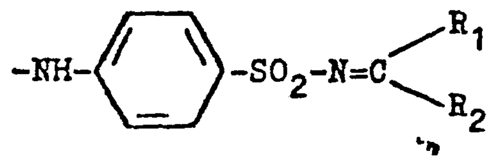

,(IIa)

in der die Reste $R_1$ und $R_2$ gleich oder verschieden sind und die Methylmercaptogruppe oder die Aminogruppe bedeuten, oder

2. eine Gruppe der allgemeinen Formel IIb

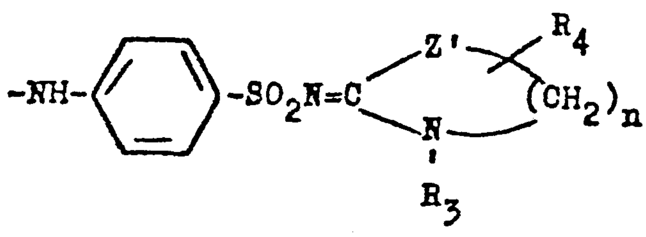

,(IIb)

in der

Z' für -O-, -S- oder $=NR_3$ und

$R_3$ für ein Wasserstoffatom oder eine Alkylgruppe mit 1 bis 3 Kohlenstoffatomen steht, die gegebenenfalls durch eine Hydroxygruppe substituiert sein kann und

$R_4$ ein Wasserstoffatom, die Hydroxy-, Hydroxymethyl- oder Methylgruppe bedeutet

und n die Zahlen 2, 3 oder 4 darstellt, wobei die Gruppen der Formeln IIa und IIb, wenn einer oder beide Reste $R_1$ und $R_2$ eine Aminogruppe bedeuten oder wenn $R_3$ Wasserstoff darstellt, auch in den folgenden tautomeren Formen der Formeln IIa' bzw. IIb' vorliegen können:

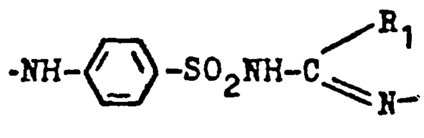 oder 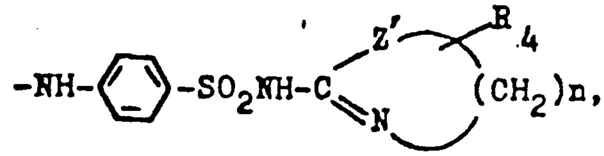

IIa' IIb'

oder

3. eine Gruppe der allgemeinen Formel

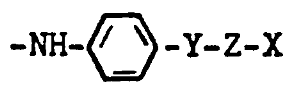

in der

Y eine Gruppe der Formel $-SO_2-NH-$, $-SO-$ oder $-SO_2-$ und

Z eine geradkettige oder verzweigte Alkylengruppe mit 1 bis 3 Kohlenstoffatomen und

X eine Hydroxy-, Aminocarbonyl-, Aminosulfonyl-, Formylamino-, Acetylamino-, Amino-, Methylsulfinyl-, Methylsulfonyl- oder eine Aminocarbonsäuregruppe

der Formel

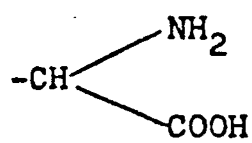

sein können,

oder es bedeutet -Y-Z-X

die Gruppe $-SO_2N(CH_2CH_2OH)_2$, die Gruppe 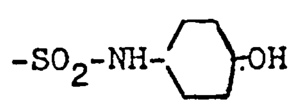

die Gruppe 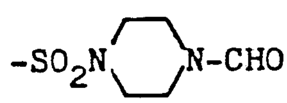 oder 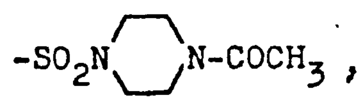 ,

die Gruppe 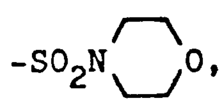, oder 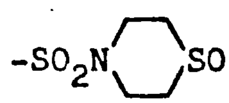 oder

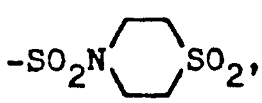, oder die Gruppe

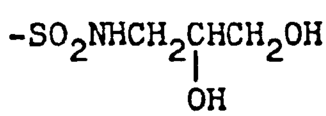 oder 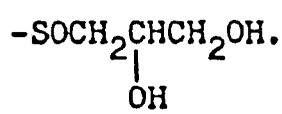.

und von deren Salzen mit anorganischen oder organischen Basen, dadurch gekennzeichnet, daß

a) eine Verbindung der allgemeinen Formel III,

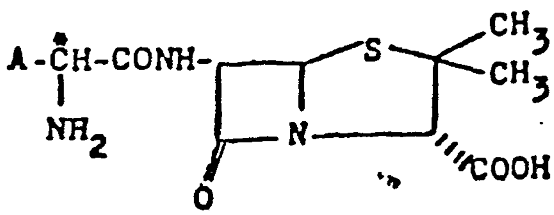

,(III)

in der A wie oben definiert ist, oder ihre Salze oder Ester mit einem Pyrimidinderivat der allgemeinen Formel IV,

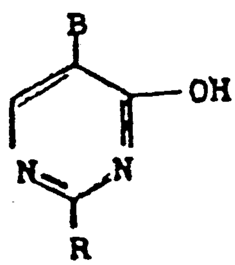

,(IV)

in der R wie oben definiert ist und B die Gruppe -NCO oder ein reaktives Derivat der Gruppe NHCOOH bedeutet, wie z. B.

die Gruppen -NHCOCl, -NHCOBr oder -NH-COO-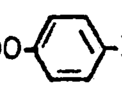-$NO_2$,

wobei die Gruppe NHCOCl besonders bevorzugt ist, oder mit Gemischen von solchen Pyrimidinderivaten der allgemeinen Formel IV, in der B teils die eine und teils die andere der vorstehend genannten Bedeutungen besitzt, in einem Lösungsmittel bei einem pH-Wert zwischen 2,0 und 9,0 bei Temperaturen zwischen -20 und +50°C, gegebenenfalls in Gegenwart einer Base, umgesetzt wird oder

2. Verfahren zur Herstellung neuer Penicilline der allgemeinen Formel I bzw. I'

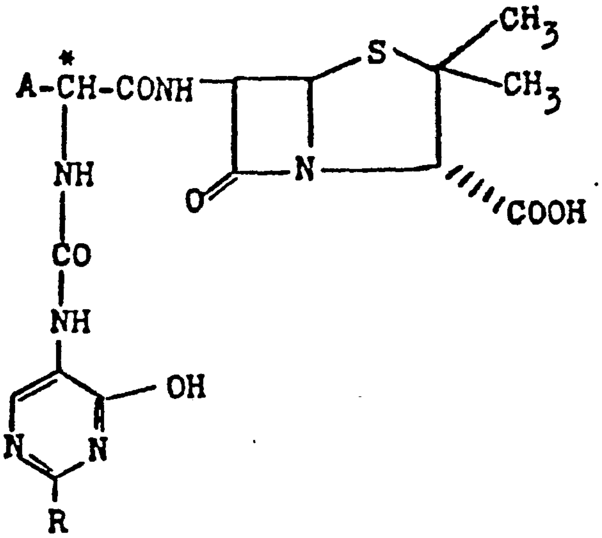

,(I)

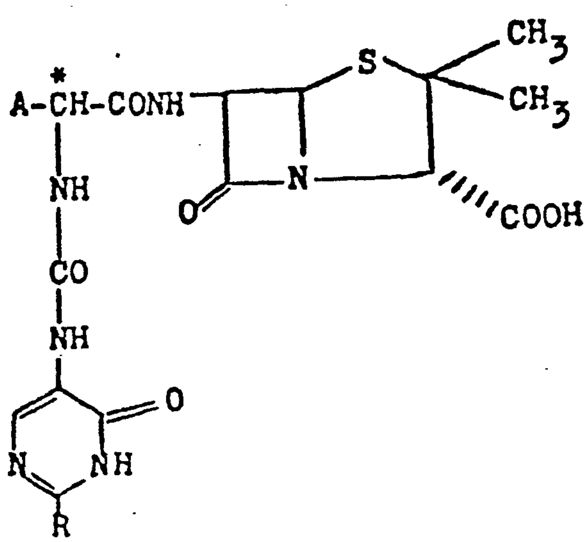

,(I')

in der A und R die folgenden Bedeutungen innehaben:

A die Phenyl-, 4-Hydroxyphenyl-, die 2-Thienyl-, die 3,4-Dihydroxyphenylgruppe

R eine Gruppe der allgemeinen Formel IIa

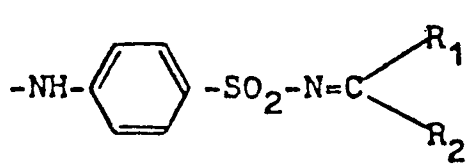

,(IIa)

b) Ureidocarbonsäuren der allgemeinen Formel V,

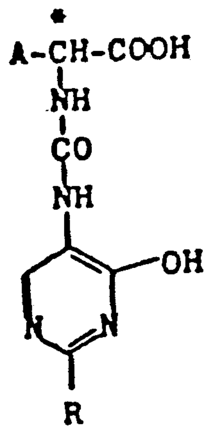

,(V)

in der A und R die oben angegebenen Bedeutungen besitzen, ihre Salze oder reaktiven Derivate, mit der 6-Aminopenicillansäure der Formel VI,

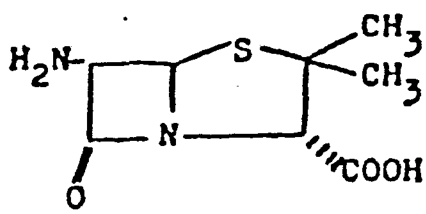

,(VI)

oder ihren anorganischen oder organischen Salzen oder Derivaten, die in die 6-Aminopenicillansäure leicht überführbar sind, in einem Lösungsmittel bei Temperaturen zwischen -40 und +40°C, gegebenenfalls in Gegenwart einer Base, umgesetzt werden und, gewünschtenfalls, so erhaltene Verbindungen der allgemeinen Formel I bzw. I' anschließend mittels anorganischer oder organischer Basen in ihre Salze übergeführt werden.

in der die Reste $R_1$ und $R_2$ gleich oder verschieden sind und folgende Bedeutung haben können:
die Methylmercaptogruppe, oder die Aminogruppe,
R kann aber auch
eine Gruppe der allgemeinen Formel IIb

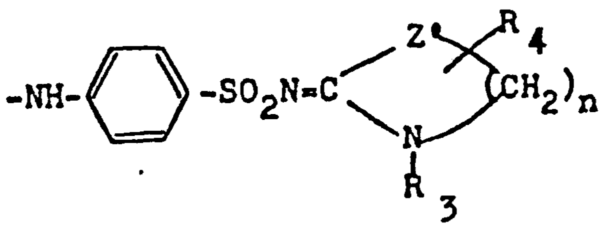
,(IIb)

darstellen, in der
Z' für -O-, -S- oder $=NR_3$ und
$R_3$ für ein Wasserstoffatom oder eine Alkylgruppe mit 1 bis 3 Kohlenstoffatomen steht, die gegebenenfalls durch eine Hydroxygruppe substituiert sein kann,
$R_4$ ein Wasserstoffatom, die Hydroxy-, Hydroxymethyl- oder Methylgruppe bedeutet
und n die Zahlen 2, 3 oder 4 darstellt,
und von deren Salzen mit anorganischen oder organischen Basen dadurch gekennzeichnet, daß

a) eine Verbindung der allgemeinen Formel III,

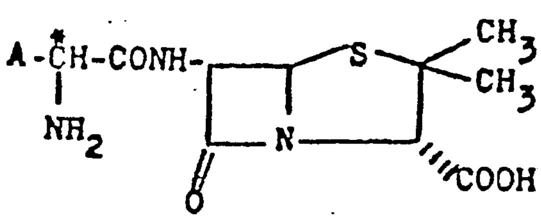
,(III)

in der A wie oben definiert ist, oder ihre Salze oder Ester mit einem Pyrimidinderivat der allgemeinen Formel IV,

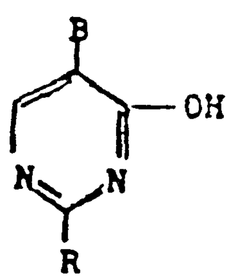
,(IV)

in der R wie oben definiert ist und B die Gruppe -NCO oder ein reaktives Derivat der Gruppe NHCOOH bedeutet, wie z.B.

die Gruppen -NHCOCl, -NHCOBr oder -NH-COO-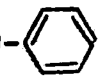-$NO_2$,

wobei die Gruppe NHCOCl besonders bevorzugt ist, oder mit Gemischen von solchen Pyrimidinderivaten der allgemeinen Formel IV, in der B teils die eine und teils die andere der vorstehend genannten Bedeutungen besitzt, in einem Lösungsmittel bei einem pH-Wert zwischen 2,0 und 9,0 bei Temperaturen zwischen -20 und +50°C, gegebenenfalls in Gegewart einer Base, umgesetzt wird oder

b) Ureidocarbonsäuren der allgemeinen Formel V,

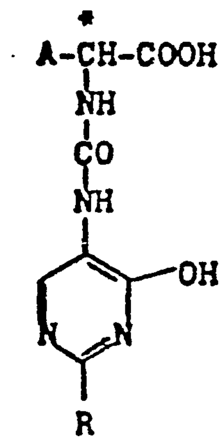

,(V)

in der A und R die oben angegebenen Bedeutungen besitzen, ihre Salze oder reaktivenDerivate, mit der 6-Amino-penicillansäure der Formel VI,

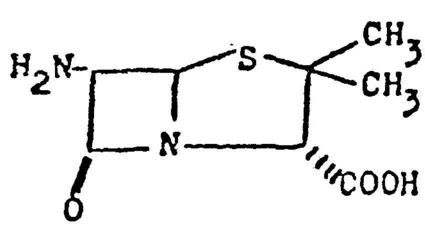

,(VI)

oder ihren anorganischen oder organischen Salzen oder Derivaten, die in die 6-Aminopenicillansäure leicht überführbar sind, in einem Lösungsmittel bei Temperaturen zwischen -40 und +40°C, gegebenenfalls in Gegenwart einer Base, umgesetzt werden und, gewünschtenfalls, so erhaltene Verbindungen der allgemeinen Formel I bzw. I' anschließend mittels anorganischer oder organischer Basen in ihre Salze übergeführt werden.

3. Verfahren zur Herstellung neuer Penicilline der allgemeinen Formel I

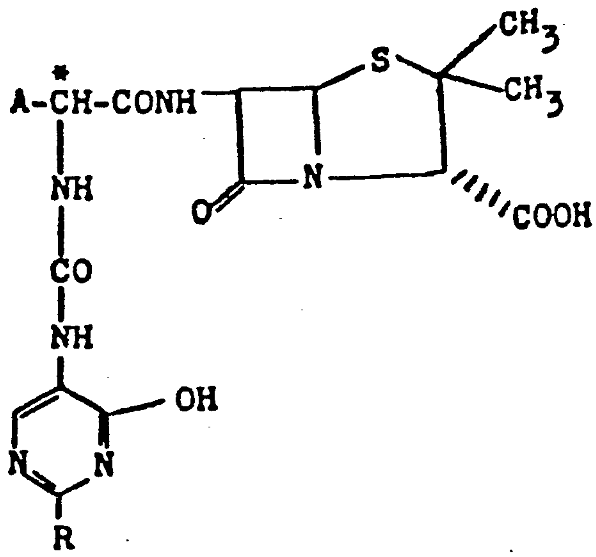

,(I)

bzw. von deren Tautomeren der allgemeinen Formel I'

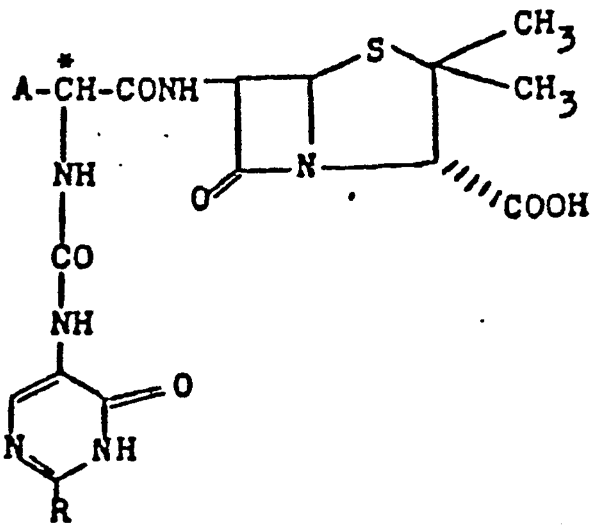
,(I')

wobei in den obigen allgemeinen Formeln I und I'
A und R die folgenden Bedeutungen innehaben:
A die Phenyl-, 4-Hydroxyphenyl-, die 2-Thienyl-, die 3,4-Dihydroxyphenylgruppe,

R eine Gruppe der allgemeinen Formel

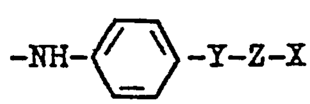

in der
Y eine Gruppe der Formel $-SO_2-NH-$, -SO- oder $SO_2-$ und

Z eine geradkettige oder verzweigte Alkylengruppe mit 1 bis 6 Kohlenstoffatomen bedeutet
und
X eine Hydroxy-, Aminocarbonyl-, Aminosulfonyl-, Formylamino-, Acetylamino-, Amino-, Methylsulfinyl-, Methylsulfonyl- oder eine Aminocarbonsäure der Formel

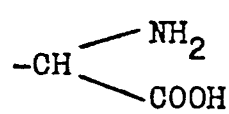

sein kann,
oder es bedeutet -Y-Z-X
die Gruppe $-SO_2N(CH_2CH_2OH)_2$

die Gruppe 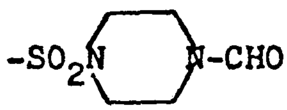 oder 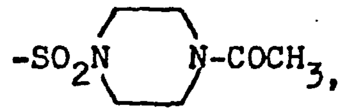,

die Gruppe 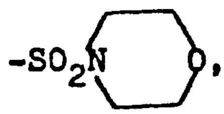, 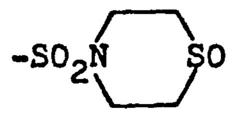 oder 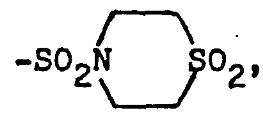,

oder die Gruppe 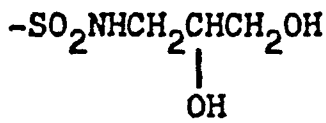 oder 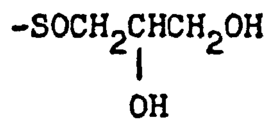

und von ihren physiologisch verträglichen Salzen mit anorganischen oder organischen Basen, dadurch gekennzeichnet, daß

a) eine Verbindung der allgemeinen Formel III,

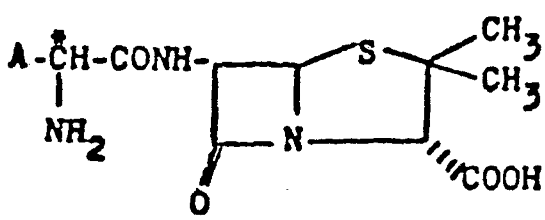 ,(III)

oder ihre Salze mit anorganischen oder organischen Basen oder ihre leicht spaltbaren Ester oder Silylierungsprodukte, in der A wie oben definiert ist, mit einem Pyrimidinderivat der allgemeinen Formel IV,

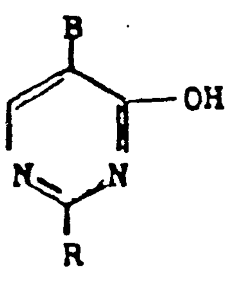 ,(IV)

in der R wie oben definiert ist und B die Gruppe -NCO oder ein reaktives Derivat der Gruppe -NHCOOH bzw. die Gruppen -NHCOCl, -NHCOBr oder -NH-COO-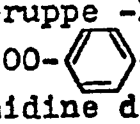-$NO_2$ bedeutet, oder mit Gemischen solcher Pyrimidine der allgemeinen Formel IV, in der B teils die eine und teils die andere der vorstehend genannten Bedeutungen besitzt, in einem Lösungsmittel und bei einem pH-Bereich zwischen 2,0 und 9,0 bei Temperaturen zwischen -20°C und +50°C umgesetzt wird,

oder

b) eine Ureidocarbonsäure der allgemeinen Formel V,

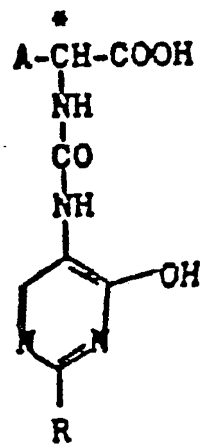

,(V)

in der A und R die oben angegebenen Bedeutungen besitzen, oder ihre Salze oder reaktiven Derivate, mit der 6-Aminopenicillansäure der Formel VI,

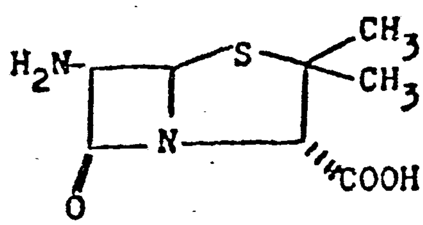

,(VI)

oder ihren anorganischen oder organischen Salzen oder Derivaten, die in die 6-Aminopenicillansäure leicht überführbar sind, wie ihre leicht abspaltbaren Ester, zwischen $-40^{o}$ und $+40^{o}C$ in Gegenwart eines Lösungsmittels und, gegebenenfalls, einer Base umgesetzt wird, gegebenenfalls das so gewonnene Produkt, welches an der Carboxylgruppe verestert ist, anschließend zu einer Verbindung der allgemeinen Formel I bzw. I' hydrolysiert oder katalytisch hydrogenolisiert wird und gewünschtenfalls anschließend die Verbindungen der allgemeinen Formel I bzw. I' in ihre physiologisch verträglichen Salze mit anorganischen oder organischen Basen überführt werden.

4. Verfahren gemäß Anspruch 1a, dadurch gekennzeichnet, daß eine Verbindung der allgemeinen Formel III, oder eines ihrer Salze mit anorganischen oder organischen Basen mit einer Verbindung der allgemeinen Formel IV

   a) in Wasser oder in mit Wasser mischbaren Lösungsmitteln in Gegenwart von Wasser in einem pH-Bereich von 6,5 bis 8,0 oder
   b) in wasserfreien Lösungsmitteln oder
   c) in einem Gemenge aus Wasser und mit Wasser nicht mischbaren Lösungsmitteln in einem pH-Bereich zwischen 6,5 und 8,0

   umgesetzt wird, oder daß eine Verbindung der allgemeinen Formel III, in der in der Carboxylgruppe das Proton durch eine Silylgruppe oder eine andere leicht abspaltbare Schutzgruppe ersetzt ist, mit einer Verbindung der allgemeinen Formel IV in einem wasser- und hydroxylgruppenfreien bzw. aprotischen Lösungsmittel, gegebenenfalls in Gegenwart einer Base, umgesetzt wird.

5. Verfahren gemäß Anspruch 1b, dadurch gekennzeichnet, daß als reaktive Derivate der Ureidocarbonsäuren der allgemeinen Formel V deren Säureanhydride, deren reaktive Ester oder reaktive Amide oder deren Säurehalogenide, als Derivate der 6-Aminopenicillansäure der Formel VI ihre Ester, vorzugsweise ihr Trimethylsilylester, Tritylester, p-Nitrobenzylester, Phenacylester oder O,N-Bis-trimethylsilylderivat verwendet werden und die Umsetzung, wenn erforderlich, in Gegenwart einer Base und/oder eines organischen Lösungsmittels und/oder eines Kondensationsmittels erfolgt, und erforderlichenfalls das so erhaltene Produkt der allgemeinen Formel I bzw. I', dessen Carboxylgruppe durch eine Schutzgruppe geschützt ist, anschließend in eine Verbindung der allgemeinen Formel I bzw. I' übergeführt wird, die die freie Carboxylgruppe besitzt.

0053693

Europäisches Patentamt

# EUROPÄISCHER RECHERCHENBERICHT

Nummer der Anmeldung

EP 81 10 8

| EINSCHLÄGIGE DOKUMENTE | | | KLASSIFIKATION DER ANMELDUNG (Int Cl ³) |
|---|---|---|---|
| Kategorie | Kennzeichnung des Dokuments mit Angabe, soweit erforderlich, der maßgeblichen Teile | betrifft Anspruch | |
| Y | EP - A - 0 016 374 (KARL THOMAE)<br>* Ansprüche *<br>-- | 1,5-8 | C 07 D 499/64<br>A 61 K 31/43//<br>C 07 D 239/48<br>D 413/12<br>D 417/12<br>D 403/12<br>D 409/12 |
| Y | EP - A - 0 006 532 (KARL THOMAE)<br>* Ansprüche *<br>-- | 1,5-8 | |
| P,Y | EP - A - 0 022 494 (KARL THOMAE)<br>* Ansprüche *<br>---------- | 1,5-8 | RECHERCHIERTE SACHGEBIETE (Int Cl ³)<br>C 07 D 499/00<br>A 61 K 31/00 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| DEN HAAG | 09-02-1982 | CHOULY |